(19)

Europäisches
Patentamt
European
Patent Office
Office européen
des brevets

(11) **EP 4 279 131 A1**

(12) # EUROPEAN PATENT APPLICATION

(43) Date of publication:
**22.11.2023 Bulletin 2023/47**

(21) Application number: **22382491.3**

(22) Date of filing: **20.05.2022**

(51) International Patent Classification (IPC):
*A61P 17/02* [(2006.01)]  *A61K 35/745* [(2015.01)]
*A61K 35/747* [(2015.01)]  *A61P 17/10* [(2006.01)]
*A61P 17/14* [(2006.01)]

(52) Cooperative Patent Classification (CPC):
(C-Sets available)
**A61P 17/14; A61K 35/745; A61K 35/747;**
**A61P 17/02; A61P 17/10**                    (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(83) **Declaration under Rule 32(1) EPC (expert solution)**

(71) Applicant: **Bioithas S.L.**
**03430 Onil Alicante (ES)**

(72) Inventor: **NAVARRO LÓPEZ, Vicente Manuel**
**03430 Alicante (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **PROBIOTIC COMPOSITIONS FOR THE TREATMENT OF ALOPECIA**

(57)    The present invention is related to probiotic compositions for use in the treatment and/or prevention of cicatrical and/or non-cicatrical alopecia and the probiotic compositions themselves comprising at least one strain of *Lactobacillus* and at least one strain of *Bifidobacterium.*

EP 4 279 131 A1

(52) Cooperative Patent Classification (CPC): (Cont.)

C-Sets
**A61K 35/745, A61K 2300/00;**
**A61K 35/747, A61K 2300/00**

**Description**

**Technical field**

The present invention is related to probiotic compositions for use in the treatment and/or prevention of cicatrical and/or non-cicatrical alopecia and the probiotic compositions themselves comprising at least one strain of *Lactobacillus* and at least one strain of *Bifidobacterium*.

**Background of the invention**

The term probiotic has been defined as "living microorganisms which, when consumed in adequate amounts, confer a health effect on the host". The earliest report on probiotics dates back to 1907, when Elie Metchnikoff described a correlation between the ingestion of lactic acid-producing bacteria in yogurt and enhanced longevity. During the past few decades, there has been renewed interest in probiotics not only regarding digestive health, but also in the management of inflammatory diseases.

Oral probiotics have been shown to improve insulin sensitivity in animal models as well as regulate the release of inflammatory cytokines in the skin through their interaction with gut-associated lymphoid tissue (1). In fact, the gut-brain-skin axis suggests a mechanism that links gastrointestinal health, influenced by interactions with oral probiotics, to the health and well-being of the skin (2). In this sense, several strains of *Lactobacillus* have been shown to have systemic anti-inflammatory effects. Studies have shown that *Lactobacillus reuteri* induces systemic anti-inflammatory cytokines, such as interleukin (IL)-10. Soluble factors from *L. reuteri* inhibit production of pro-inflammatory cytokines and culture supernatants of murine-derived *L. reuteri* 6798 inhibit tumor necrosis factor (TNF) production of activated macrophages (3).

Several strains of *Lactobacillus* also demonstrate anti-inflammatory properties. The addition of *Lactobacillus paracasei* NCC2461 has been shown to inhibit neutrogenic inflammation in a skin model, and the addition of *L. paracasei* NCC2461 to lymphocyte culture has been shown to strongly inhibit the proliferative activity of CD-4+ T-cells in a dose-dependent manner and to induce the anti-inflammatory cytokines IL-10 and TGF-beta. Mice who consumed the probiotic for 7 days demonstrated a significantly higher antibody response and in vivo T-cell-mediated immune response, indicating *L. paracasei* affects both B-and T-cell function. Similarly, mice treated with *L. casei* had an increased ability to produce IL-10 and promote T-regulatory cell function. An increase in T-regulatory cells suggests that this probiotic may help to balance the immune system's response to stimuli (1). Consequently, certain probiotic strains may potentially boost appropriate immune responses, for example to a harmful pathogenic threat, while dampening the unnecessary immune responses seen in chronic inflammatory states.

Overall, probiotics modulate the development of the immune system, often shifting the immune response toward regulatory and anti-inflammatory conditions. This ability of probiotics to modify chronic inflammatory states suggests that probiotics may have a role in treating chronic inflammatory conditions, ranging from inflammatory bowel disease to reactive airway disease to acne, rosacea, atopic dermatitis, and photoaging (4).

Furthermore, the involvement of the microbiome in many different scalp conditions has been investigated over the years. Studies on the role of the scalp microbiome in specific diseases, such as those involving hair growth alterations like non-cicatricial alopecia such as for example androgenetic alopecia (AGA) and alopecia areata (AA) and cicatricial alopecia, such as lichen planopilaris, are of major importance.

It has been previously described that the intestinal microbiota and the skin microbiota play a fundamental role in several skin diseases, and the microbial composition has been altered in these diseases (5). Some studies have described alterations in the microbiome of the scalp and hair follicles, identifying alterations in associated diseases (6). However, there is still little information available on the association between the microbial composition of the scalp and hair-associated diseases.

An increase in anaerobic gram-positive and gram-negative bacterial genera was identified in a study on hidradenitis suppurativa compared to healthy individuals. In addition, a positive association between the relative abundance of anaerobes and the severity of the disease was postulated (7).

Primary cicatricial alopecias (PCA) represent a challenging group of disorders that result in irreversible hair loss from the destruction and fibrosis of hair follicles. Cicatricial alopecias (CA) describes multiple subtypes of hair loss in which the hair follicle is destroyed by an unknown inflammatory mechanism.

Alopecia areata (AA) is a common, inflammatory, non-scarring type of hair loss with a prevalence of 0.2% affecting 2% of the population at some point in their life. It develops more frequently in children and young adults between the third and fourth decades of life, although it can manifest at any age, without gender predominance. Family incidence varies from 10-20% and 90% of cases affect the scalp, although it can appear in other hairy areas, such as for example the eyebrows. In 30% of patients an autoimmune disease is associated. This type of alopecia can have a significant impact on the life of the patient, since it negatively influences the formation of the image and self-esteem of the body, which makes it play an important role in the socialization process, causing in many cases an inefficient functioning in the social environment and a decrease in the quality of life.

The etiopathogenesis of AA is autoimmune, although other factors such as genetics and certain environmental factors that act as triggers participate. The precise mechanisms of the appearance of this are not completely known. There is some clinical and experimental evidence indicating that AA is the demonstration of an autoimmune attack against the hair follicles that causes an inflammatory condition of the hair follicle, causing an increase in the production and proliferation of T cells and autoreactive antibodies. However, the specific factors involved in such autoimmunity in AA are still unknown.

The diagnosis is fundamentally clinical, supported by the findings observed by trichoscopy, although occasionally a skin biopsy may be necessary to confirm the diagnosis. It is recommended to apply the SALT scale (Severity of Alopecia Tool), which evaluates the surface of the scalp affected by AA to assess the severity of alopecia. The scale defines 6 degrees of extension, in ascending order of severity.

The AA treatment should be individualized. It should be noted that on many occasions the alteration of the natural course of the disease is not achieved. Conservative treatment should be considered in children and mild forms. Treatments currently used for AA are the use of topical or intralesional corticosteroids, topical minoxidil, contact immunotherapy, photochemotherapy, excimer laser, topical anthralin 1%, systemic corticoids, anti-JAK therapy, thermodynamic therapy, calcineurin inhibitors and, sulfasalazine. Newer therapeutic targets include immunomodulators, Tregs, Platelet-rich plasma therapy and Statins.

Despite there being more and more evidence showing that the human microbiome plays a key role in human health and disease, no study has been conducted to analyse an eventual involvement of the microbiome, and more specifically the human microbiota and its modulation on the AA pathogenesis. Rebello et al. shows a recent case study describing hair growth in two patients with AA after faecal microbiota transplantation, which may suggest a possible role of the gut microbiota in the disease mechanism (8). One study of microbiota profiling of the scalp described an alteration in patients with alopecia areata (9). Alpha-diversity (Shannon diversity index) was significantly higher in the scalp of alopecia areata subjects than in the control group, and an increase of *Propionibacterium* in AA subjects alongside a general decrease of *Staphylococcus* were detected (9).

So far, no treatment options based on probiotics have been proposed for the treatment of alopecia. Due to the various benefits of such a treatment, there is a need to provide probiotic compositions that deliver a significant improvement in the overall treatment of alopecia, in particular a significant improvement according to well-known and accepted grading systems such as the SALT (Severity of Alopecia tool) Scale.

The inventors therefore have carried out a study to assess the possible involvement of the intestinal and skin microbiota in the unclear AA pathogenesis, which lead to a successful alternative treatment option for patients suffering from the current treatment limitations as further described herein.

## Brief description of the figures

**Figure 1:** Depiction of the SALT Scale for the assessment of Alopecia.

**Figure 2:** This figure shows the consort diagram of the study.

**Figure 3:** This figure shows the Alopecia Areata trichoscopy signs used in the assessment. A) Trichoscopy signs of AA and their relationship with disease activity. B) AA trichoscopy signs.

**Figure 4:** This figure shows the Dermatology Life Quality Index (DLQI) Questionnaire to assess the impact that Alopecia Areata has on quality of life. Each question answered with "Very much" is counted with 3 points, "Much" with 2, "Slightly" with 1, and "Not at all" as 0.

**Figure 5:** This figure shows the significant reduction of certain bacterial genera after treatment with the probiotic composition. A) *Bifidobacterium;* B) *Escherichia/Shigella;* C) *Rhodococcus;* D) *Erysipelothrix;* E) *Parabacteroides.* P- value: <0.01 (**); <0.05 (*). TTO1_V0-1: Probiotic - baseline (week 0), TTO1_V4: Probiotic - final (week 24), TTO2_V0-1: Placebo - baseline (week 0), TTO2_V4: Placebo - final (week 24).

## Summary of the invention

The present invention relates in one aspect to a probiotic composition for use in the treatment and/or prevention of alopecia, preferably cicatrical and/or non-cicatrical alopecia, wherein the probiotic composition comprises at least one strain of *Lactobacillus* and at least one strain of *Bifidobacterium.*

In one embodiment of the probiotic composition for use according to present invention

    i. the at least one strain of *Lactobacillus* is selected from *L. rhamnosus, L. casei, L. paracasei* and *L. reuteri,* preferably wherein the strain is *L. rhamnosus,* most preferred *L.rhamnosus* CECT 30580; and/or

    ii. the at least one strain of *Bifidobacterium* is selected from B. *animalis subspecies lactis* and *B. longum,* preferably wherein the strain is B. *longum,* most preferred B. *longum* CECT 30616.

In a further embodiment of the probiotic composition for use according to present invention

    (i) the cicatrical alopecia is selected from Lichen planopilaris (LPP), Lymphocytic cicatrical alopecia, such as Central centrifugal cicatricial alopecia (CCCA), Discoid lupus erythematous (DLE), Frontal fibrosing alopecia (FFA), Graham-Little syndrome (GLS), Pseudopelade of Brocq Follicular mucinosis (FM) and Keratosis follicularis spinulosa decalvans (KFSD), Neutrophilic cicatrical alopecia, such as Folliculitis decalvans (FD) and Dissecting cellulitis of the scalp (DCS), Mixed cicatrical alopecia, such as Acne keloidalis (AK), Acne necrotica (AN), Erosive pustular dermatosis of the scalp (EPDS), or any combinations thereof; and/or

    (ii) the non-cicatrical alopecia is selected from Androgenetic Alopecia (AGA) and Alopecia Areata (AA).

In a further embodiment of the probiotic composition for use according to present invention, the probiotic composition is a pharmaceutical composition or a nutritional composition.

In one embodiment of the probiotic composition for use according to present invention, the pharmaceutical composition comprises a pharmaceutically acceptable carrier and/or an excipient.

In a further embodiment of the probiotic composition for use according to present invention, the pharmaceutical composition is formulated for administration in a liquid or a solid formulation. In one aspect if the invention the solid formulation is selected from the group consisting of tablets, lozenges, sweets, chewable tablets, chewing gum, capsules, sachets, powders, granules, coated particles and/or tablets, gastro-resistant tablets and/or capsules and dispersible strips and/or films. In a further aspect of the invention the liquid formulation is selected from the group consisting of oral solutions, suspensions, emulsions and syrups.

In a further aspect, the probiotic composition for the use according to present invention is a food or a nutritional supplement. In one aspect the said food is selected from the group consisting of fruit or vegetable juices, ice cream, milk, yogurt, cheese, fermented milk, milk powder, infant formula, cereals, baked goods, milk-based products, meat products and beverages. In a further aspect the said nutritional supplement is selected from the group consisting of Biotin, Lutein, Selenium, Zinc, Copper, or combinations thereof. Preferred are of Biotin, Lutein, Zinc, or combinations thereof.

The probiotic composition for the use according to present invention, wherein the composition further comprises further microorganisms selected from the group consisting of *Lactobacillus sp., Bifidobacterium sp., Kluyveromyces sp., Bacillus sp.,* and combinations thereof.

In one embodiment of the probiotic composition for use according to present invention, the probiotic composition is provided as a single dosage form.

In one aspect of this embodiment the total concentration of microorganisms of the strains *Lactobacillus* and *Bifidobacterium* in the single dosage form is between about $10^3$ and $10^{15}$ cfu/dose, preferably between about $10^6$ and $10^{12}$ cfu/dose, more preferably about $10^9$ cfu/dose.

In a further aspect the *Lactobacillus* concentration is at least between 30% and 70%, at least between 40% and 60%, at least between 45% and 55% with respect to the total concentration of microorganisms present in the composition.

In yet a further aspect the *Bifidobacterium* concentration is at least between 30% and 70%, at least between 40% and 60%, at least between 45% and 55% with respect to the total concentration of microorganisms present in the composition.

In one further embodiment the present invention relates to a probiotic composition comprising a mixture of at least two probiotic strains, wherein at least one probiotic strain is a strain of Lactobacillus and at least one probiotic strain is a strain of Bifidobacterium, wherein

    i. the at least one strain of *Lactobacillus* is selected from *L. rhamnosus, L. casei, L. paracasei* and *L. reuteri,* preferably wherein the strain is *L. rhamnosus,* most preferred *L.rhamnosus* CECT 30580; and/or

    ii. the at least one strain of *Bifidobacterium* is selected from B. *animalis subspecies lactis* and *B. longum,* preferably wherein the strain is B. *longum,* most preferred B. *longum* CECT 30616.

### Description of the invention

The present invention relates in one aspect to a probiotic composition for use in the treatment and/or prevention of alopecia, preferably cicatrical and/or non-cicatrical alopecia, wherein the probiotic composition comprises at least one strain of *Lactobacillus* and at least one strain of *Bifidobacterium.*

Alopecia is known as a primary hair loss disorder. The two main types of alopecia are non-cicatricial alopecia, also known as non-scarring alopecia, and cicatrical alopecia, also known as scarring alopecia. Non-cicatricial types of alopecia refer to hair loss due to changes in the hair cycle, hair follicle size, hair breakage or a combination of these changes, with preservation of the hair follicle. Cicatricial alopecias refer to forms of hair loss in which hair follicles are destroyed owing to inflammation or, rarely, malignancy (such as cutaneous lymphoma). Affected skin shows loss of follicular ostia, the openings of the hair follicle through which the hair fibre emerges through the skin.

Primary cicatricial alopecias (PCA) represent a challenging group of disorders that result in irreversible hair loss from the destruction and fibrosis of hair follicles. Cicatricial alopecias (CA) describes multiple subtypes of hair loss in which the hair follicle is destroyed by an unknown inflammatory mechanism.

In one embodiment of the probiotic composition for use according to present invention the type of cicatrical alopecia to be treated is selected from for example Lichen planopilaris (LPP), Lymphocytic cicatrical alopecia, such as Central centrifugal cicatricial alopecia (CCCA), Discoid lupus erythematous (DLE), Frontal fibrosing alopecia (FFA), Graham-Little syndrome (GLS), Pseudopelade of Brocq Follicular mucinosis (FM) and Keratosis follicularis spinulosa decalvans (KFSD), Neutrophilic cicatrical alopecia, such as Folliculitis decalvans (FD) and Dissecting cellulitis of the scalp (DCS), Mixed cicatrical alopecia, such as Acne keloidalis (AK), Acne necrotica (AN), Erosive pustular dermatosis of the scalp (EPDS), or any combinations thereof.

In a further embodiment the non-cicatrical alopecia is selected from Androgenetic Alopecia (AGA) and Alopecia Areata (AA). A preferred type of non-cicatrical alopecia is Alopecia areata (AA), which is a common, inflammatory, nonscarring type of hair loss.

It is to be understood that the above listed alopecia types are not limiting. Other types of alopecia known in the art are also envisaged for being treated as disclosed in present invention.

In the present invention the term "probiotic composition", as used in any of the embodiment of present invention, is understood as a composition comprising microorganisms which, when ingested, interact with the individual's metabolism and produce a beneficial effect in it. In the present invention, the probiotic composition comprises a mixture of at least one strain of *Lactobacillus* and at least one strain of *Bifidobacterium.*

In one embodiment the at least one strain of *Lactobacillus* is selected from *L. casei, L. rhamnosus, L. paracasei* and *L. reuteri*. The preferred Lactobacillus is *L. rhamnosus,* the most preferred is *L. rhamnosus* CECT 30580. *L. rhamnosus* is a bacterium commonly used as a probiotic, found mostly in yogurt and other dairy products, including infant formula. *L. rhamnosus* is classified as follows: Domain: Bacteria, Phylum: Firmicutes, Class: Bacilli, Order: Lactobacillales, Family: Lactobacillaceae, Genus: *Lactobacillus,* Species: *L. rhamnosus*

In a further embodiment the at least one strain of Bifidobacterium is selected from B. *animalis subspecies lactis* and *B. longum,* preferably wherein the strain is B. *longum,* most preferred being B. *longum* CECT 30616. *Bifidobacterium* is a genus of gram-positive, nonmotile, often branched anaerobic bacteria that are ubiquitous inhabitants of the gastrointestinal tract. The scientific classification of *B.longum* is: Domain: Bacteria, Phylum: Actinomycetota, Order: Bifidobacteriales, Family: Bifidobacteriaceae, Genus: *Bifidobacterium,* Species: *B. longum.*

The probiotic composition for use according to present invention can be a pharmaceutical composition, or a nutritional composition.

In a particular embodiment, the pharmaceutical composition of the invention further comprises a pharmaceutically acceptable carrier and/or excipient.

The term "excipient" refers to a substance that helps the absorption of any components or compounds of any of the probiotic compositions of the invention, namely, of strains of the invention, or stabilizes the components or compounds and/or assists the preparation of the pharmaceutical composition in the sense of giving it consistency or flavors to make it more pleasant. Thus, the excipients may have the function, by way of example but not limited thereto, of binding the components (for example, starches, sugars or cellulose), sweetening, colouring, protecting the active ingredient (for example, to insulate it from air and/or moisture), filling a pill, capsule or any other presentation or a disintegrating function to facilitate dissolution of the components, without excluding other excipients not listed in this paragraph. Therefore, the term "excipient" is defined as that material that included in the galenic forms, is added to the active ingredients or their associations to enable their preparation and stability, modify their organoleptic properties or determine the physicochemical properties of the pharmaceutical composition and its bioavailability. The "pharmaceutically acceptable" excipient must allow the activity of components or compounds of the pharmaceutical composition, that is, be compatible with the strains of the invention.

The "galenic form" or "pharmaceutic form" is the configuration to which the active ingredients and excipients are adapted to provide the pharmaceutical composition or drug of the invention. It is defined by the combination of the form in which the pharmaceutical composition is presented by the manufacturer and the form in which it is administered.

The "vehicle" or "carrier" is particularly an inert substance. Carrier functions are to facilitate the incorporation of other components or compounds, allow better dosage and administration and/or give consistency and form to the pharmaceutical composition. Therefore, the carrier is a substance used in the drug to dilute any of the components or compounds of the pharmaceutical composition of the present invention to a given volume or weight; or that even without diluting these components or compounds, it is able to allow better dosage and administration and/or give consistency and form to the drug. When the presentation is liquid, the pharmaceutically acceptable carrier is the diluent. The carrier can be natural or unnatural. Examples of pharmaceutically acceptable carriers include, without being limited thereto, water, salt solutions, alcohol, vegetable oils, polyethylene glycols, gelatin, lactose, starch, amylose, magnesium stearate, talc, surfactants, silicic acid, viscous paraffin, perfume oil, monoglycerides and diglycerides of fatty acids, fatty acid esters petroetrals, hydroxymethylcellulose, polyvinylpyrrolidone and the like.

Furthermore, the excipient and the carrier must be pharmacologically acceptable, i.e., the excipient and the carrier are permitted and evaluated so as not to cause damage to the subject to whom it is administered.

In each case the presentation of the pharmaceutical composition will be adapted to the type of administration used. Thus, the composition may be presented in the form of solutions, suspensions, or any other form of clinically permissible administration and in a therapeutically effective amount. The pharmaceutical composition can thus be formulated into solid, semisolid or liquid preparations, such as tablets, capsules, powders (such as those derived from lyophilization (freeze-drying) or air-drying), granules, solutions, suppositories, gels or microspheres.

In one embodiment of present invention, the pharmaceutical composition is formulated for administration in a liquid or a solid formulation.

In one aspect of the invention the solid formulation is selected from the group consisting of tablets, lozenges, sweets, chewable tablets, chewing gum, capsules, sachets, powders, granules, coated particles and/or tablets, gastro-resistant tablets and/or capsules and dispersible strips and/or films.

In a further aspect of the invention the liquid formulation is selected from the group consisting of oral solutions, suspensions, emulsions and syrups.

In one embodiment, the pharmaceutical composition is in the form of a soft gel capsules.

Likewise, various systems are known that can be used for sustained-release administration of any of the probiotic, pharmaceutical or nutritional compositions of the invention, including, for example, the encapsulation in liposomes, microbubbles, microparticles or microcapsules and the like. The suitable sustained-release forms as well as materials and methods for their preparation are well known in the state of the art. Thus, the orally administrable form of any of the probiotic compositions of the invention is in a sustained-release form further comprising at least one coating or matrix. The sustained release coating or matrix includes, without limitation, natural semisynthetic or synthetic polymers, water-insoluble or modified, waxes, fats, fatty alcohols, fatty acids, natural, semisynthetic or synthetic plasticizers or a combination of two or more of the same. Enteric coatings can be applied using conventional processes known to those skilled in the art.

In addition to what has been described above, the present invention also encompasses the possibility that any of the probiotic compositions, probiotic pharmaceutical or nutritional compositions of the present invention (as reflected in any aspect or embodiment described throughout the present specification) may be administered to a subject together with other components or compounds, although these are not part of the probiotic compositions, probiotic pharmaceutical or nutritional compositions of the present invention. In this sense, in the event that the composition of the invention is formulated as a nutritional composition, said nutritional composition may be a food or be incorporated into a food or food product intended for both human and animal consumption. Thus, in a particular embodiment, the nutritional composition is selected from a food, which may be a food for specific nutritional purposes or medicinal food, and a nutritional supplement.

In the present invention, the term "nutritional composition" refers to that food, which regardless of providing nutrients to the subject who consumes it, beneficially affects one or more functions of the body, so as to provide better health and wellness. In the present invention, said nutritional composition is intended to ease, reduce, treat and/or prevent alopecia in general and specifically cicatricial and non-cicatricial alopecia, most preferred non-cicatricial alopecia such as alopecia areata.

The term "supplement", synonymous with any of the terms "dietary supplement", "nutritional supplement", "food supplement", or "alimentary supplement" or "alimentary complement" refers to products or preparations whose purpose is to supplement the normal diet consisting of sources of concentrated nutrients or other substances with a nutritional or physiological effect. In the present invention, the "substance" which has a nutritional or physiological effect on the individual when the alimentary complement is ingested are the microorganisms *Lactobacillus,* preferably *L.rhamnosus,* most preferred *L.rhamnosus* CECT 30580 and/or *Bifidobacterium,* preferably *B.longum,* most preferred *B.longum* CECT 30616, which are part of any of the compositions of the present invention. The food supplement may be in single or combined form and be marketed in dosage form, i.e. in capsules, pills, tablets and other similar forms, sachets of powder, ampoules of liquids and drop dispensing bottles and other similar forms of liquids and powders designed to be taken in a single amount.

There is a wide range of nutrients and other elements that may be present in alimentary complements including, among others, vitamins, minerals, amino acids, essential fatty acids, fibre, enzymes, plants and plant extracts. Since their role is to complement the supply of nutrients in a diet, they should not be used as a substitute for a balanced diet and intake should not exceed the daily dose expressly recommended by the doctor or nutritionist. The probiotic composition can also be part of the so-called "food for special groups", i.e. foods that meet specific nutritional needs.

In a further aspect of the present invention the composition further comprises a supplement selected from the group consisting of Biotin, Selenium, Zinc, Copper, Lutein, or combinations thereof.

Examples of foods that may comprise the compositions or probiotic compositions of the invention include, but are not limited to, feed, dairy products, vegetable products, meat products, snacks, chocolates, drinks, baby food, cereals, fried foods, industrial bakery products and biscuits. Examples of milk products include, but are not limited to, products derived from fermented milk (for example, but not limited to, yogurt or cheese) or non-fermented milk (for example, but not limited to, ice cream, butter, margarine or whey). The vegetable product is, for example, but not limited to, a cereal in any form of presentation, fermented (for example, soy yogurt, oat yogurt, etc.) or unfermented, and a snack. The beverage may be, but is not limited to, non-fermented milk. In a particular embodiment, the food product or food is selected from the group consisting of fruit or vegetable juices, ice cream, infant formula, milk, yogurt, cheese, fermented milk, powdered milk, lyophilized or air-dried products (suitable for reconstitution with a liquid vehicle), cereals, baked goods, milk-based products, meat products and beverages.

In a further aspect, wherein the probiotic composition for the use according to present invention is a food said food is selected from the group consisting of fruit or vegetable juices, ice cream, milk, yogurt, cheese, fermented milk, milk powder, infant formula, cereals, baked goods, milk-based products, meat products and beverages.

As understood by those skilled in the art, any of the compositions or probiotic compositions of the invention may be formulated as forming part of personal care products or cosmetic products to be administered to the subject e.g. topically. Therefore, in a particular embodiment, any of the compositions or probiotic compositions of the invention can be a personal care product or a cosmetic product.

Non-limiting examples of personal care products include bar soap, liquid soap (e.g., hand soap), hand sanitizer (including rinse off and leave-on alcohol based and aqueous-based hand disinfectants), cotton swabs and pads, shaving cream, talcum powder, toiled paper, preoperative skin disinfectant, wet paper, cleansing wipes, disinfecting wipes, body wash, acne treatment products, antifungal diaper rash cream, antifungal skin cream, shampoo, conditioner, cosmetics deodorant, antimicrobial creams, body lotion, hand cream, topical cream, aftershave lotion, skin toner, oral hygiene products, and sunscreen lotion. The compositions of the invention may also be applied to wound care items, such as, but not limited to, wound healing ointments, creams, and lotions, wound coverings, burn wound cream, bandages, tape, and steri-strips, and medical articles such as medical gowns, caps, face masks, and shoe-covers, surgical drops, etc. In one particular embodiment, the personal care product is a topical cream or gel.

Additionally, any of the compositions, probiotic compositions, pharmaceutical or nutritional compositions of the present invention (as reflected in any aspect or embodiment described throughout the present specification), may comprise other microorganisms in addition to *Lactobacillus,* preferably *L.rhamnosus,* most preferred *L.rhamnosus* CECT 30580 and *Bifidobacterium,* preferably *B.longum,* most preferred *B.longum* CECT 30616. Thus, in a particular embodiment, any of the compositions, probiotic, pharmaceutical or nutritional compositions of the present invention (as reflected in any aspect or embodiment described throughout the present specification) may further comprise one or more microorganism selected, among others, from the following group: *Lactobacillus sp., Bifidobacterium sp., Kluyveromyces sp., Bacillus sp., Streptococcus sp.* and *Saccharomyces sp.* and combinations thereof. Preferred are microorganisms selected from *Bifidobacterium sp.,* such as B. *animalis subspecies lactis* and B. *longum, Lactobacillus sp.,* such as *L. casei, L. rhamnosus, L. paracasei* and *L. reuteri, Kluyveromyces sp.,* such as *K. marxianus, Bacillus sp.*, such as B. *subtilis,* and combinations thereof.

In one embodiment the probiotic composition is provided as a single dosage form. In one aspect of this embodiment the total concentration of microorganisms of the strains of *Lactobacillus,* preferably *L.rhamnosus,* most preferred *L.rhamnosus* CECT 30580 and of *Bifidobacterium,* preferably *B.longum,* most preferred *B.longum* CECT 30616 in the single dosage form is between about $10^3$ and $10^{15}$ cfu/dose, preferably between about $10^6$ and $10^{12}$ cfu/dose, more preferably about $10^9$ cfu/dose. As shown in Example 1, the composition under study contained probiotic strains at concentrations equal to or greater than $1\times10^9$ cfu/dose.

In one further embodiment the total concentration of microorganisms of the strains of *L.rhamnosus,* preferably *L.rhamnosus* CECT 30580 and/or of *B.longum,* preferably *B.longum* CECT 30616 in the single dosage form is at least about $10^6$, at least about $10^7$ at least about $10^8$ at least about $10^9$ cfu/dose. In another particular embodiment, the dose of administration of microorganisms *L.rhamnosus* CECT 30580 and/or preferably *B.longum* CECT 30616 in the composition is between $10^3$ and $10^{15}$, preferably between $10^6$ and $10^{12}$ cfu/day, particularly $10^9$ cfu/day. In a preferred embodiment, the administration regime is once daily.

In another particular embodiment of any of the probiotic pharmaceutical or nutritional compositions of the present invention (as reflected in any aspect or embodiment described throughout the present specification), the concentration of *Lactobacillus,* preferably *L.rhamnosus,* most preferred *L.rhamnosus* CECT 30580 is at least thirty percent (30%) with respect to the total concentration of microorganisms present in the composition, particularly at least 31%, 32%, 33%, 34% or 35% relative to the total concentration of microorganisms present in any of the compositions of the invention. In another even more particular embodiment, the concentration of *Lactobacillus,* preferably *L.rhamnosus,* most preferred *L.rhamnosus* CECT 30580 is at least 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 65%, 70%, 75% or 80%, preferably at least 40%, 41%, 42%, 43%, 44%, 45%, 46%, 47%, 48%, 49%, 50%, 51%, 52%, 53%, 54%, 55%, most preferred at least 45%, 46%, 47%, 48%, 49%, 50% with respect to the total amount of microorganisms present in any of the compositions of the invention.

In a further aspect the *Lactobacillus,* preferably *L.rhamnosus,* most preferred *L.rhamnosus* CECT 30580 concentration is at least between 30% and 70%, at least between 35% and 65%, at least between 40% and 60%, at least between 45% and 55% with respect to the total concentration of microorganisms present in the composition. In one preferred aspect the concentration is 50% with respect to the total concentration of microorganisms present in the composition.

In yet a further aspect the *Bifidobacterium,* preferably *B.longum,* most preferred *B.longum* CECT 30616 concentration is at least between 30% and 70%, at least between 35% and 65%, at least between 40% and 60%, at least between 45% and 55% with respect to the total concentration of microorganisms present in the composition. In one preferred aspect the concentration is 50% with respect to the total concentration of microorganisms present in the composition.

In one further embodiment the present invention relates to a probiotic composition comprising a mixture of at least two probiotic strains, wherein at least one probiotic strain is a strain of *Lactobacillus* and at least one probiotic strain is a strain of *Bifidobacterium.*

In one embodiment of the probiotic composition according to present invention

i. the at least one strain of *Lactobacillus* is selected from *L. rhamnosus, L. casei, L. paracasei* and *L. reuteri,* preferably wherein the strain is *L. rhamnosus,* most preferred *L.rhamnosus* CECT 30580; and/or
ii. the at least one strain of *Bifidobacterium* is selected from B. *animalis subspecies lactis* and *B. longum,* preferably wherein the strain is B. *longum,* most preferred B. *longum* CECT 30616.

It is to be understood that the probiotic composition *per se* of present invention can comprise any of the features as described above for the embodiments of the probiotic composition for use in the treatment of alopecia as described herein above.

As can be seen from the results in Example 1 the overall condition of the probiotic group improved in a clinically relevant manner.

Looking at the activity variable, 5/9 (55.5%) of the patients in the probiotic group improved between the initial and final visits, while 5/10 (50%) in the placebo group improved (see Table 5). The mean score difference in the probiotic group was -5.78 points, versus -3.00 in the placebo group (Table 6).

For the capillary repopulation variable, 5/9 (55.5%) of the patients in the probiotic group improved between the initial and final visits, while only 3/10 (30%) in the placebo group improved (Table 5). The mean score difference in the probiotic group was +4.40 points, versus +2.02 in the placebo group (Table 6).

Regarding the inactivity variable, 6/9 (66.7%) of the patients in the probiotic group improved between the initial and final visit, while in the placebo group 4/10 (40%) improved (Table 5). The mean score difference in the probiotic group was +4.58 points, versus +2.26 in the placebo group (Table 6).

Furthermore, the results showed that in the number of plaques variable, 5/9 (55.5%) of the patients in the probiotic group decreased the number of plaques from start to end, while in the placebo group the improvement was only seen in 3/10 (30%) (Table 7). No differences were observed in the mean improvement in the number of plaques per group (Table 8).

Regarding the AA category, no relevant differences were observed. Most of the patients in both groups maintained the initial typology at the end of the study (Table 9).

Strikingly, in the SALT scale variable of skin surface affected by alopecia, 4/9 (44.4%) of the patients in the probiotic group improved the score between the initial and final visit, while in the placebo group this improvement was seen only in 2 /10 (20%) (Table 10).

The patient adherence to treatment was around 83 % in both groups, showing that the way of administration is well accepted and convenient. Furthermore, the product was shown to be safe, with no attributable adverse events reported in the probiotic group (Table 14).

As can be seen in Example 2, the composition of the microbiota of the skin taken from the treated patients was strikingly changed reducing the amount of anaerobic and facultative anaerobic strains in the microbiome. All bacterial genera that have been reduced in the skin microbiota after the probiotic treatment of present invention with a greater improvement of clinical signs of alopecia areata compared with placebo are anaerobes or facultative anaerobes, such as *Bifidobacterium, Erysipelothrix, Paraprevotella, Rhodococcus, Anaerobacillus, Escherichia-Shigella, Kineothrix, Parabacteroides, Lachnoclostridium, Alkalihalobacillus and Mediterraneibacter.* The probiotic composition of present invention therefore provides for a surprising effect on the skin microbiome by reducing the amount of anaerobic or facultative anaerobic bacteria. On the other hand, these results together with the positive effect on alopecia areata as shown in Example 1, indicate that there could be an association between a greater presence of these genera in the skin microbiota of patients with alopecia areata and the severity of the disease.

Based on these results, it can be concluded that the present invention provides a novel and improved treatment of alopecia with a probiotic composition comprising the combination of at least two strains, in particular, bacterial strains pertaining to the species *Lactobacillus* and pertaining to the species *Bifidobacterium* as further described herein above.

The probiotic composition can furthermore be used for reducing anaerobic and facultative anaerobic genera on the skin.

In a preferred embodiment of the present invention the probiotic composition is administered orally to a subject in need of treatment.

In another particular embodiment, any of the compositions of the invention are administered to a subject through the diet.

In another particular embodiment, any of the compositions of the invention are administered to a subject via oral administration.

In one embodiment of present invention the administration of the probiotic composition to a subject leads to a reduction of anaerobic and facultative anaerobic bacterial genera on the skin.

In a further embodiment the administration of the probiotic composition to a subject provides for an improved skin microbiome.

In the present invention the term "subject" is equivalent to the term "individual", so both terms can be used interchangeably herein. "Subject" means, in addition to any individual, any animal belonging to any species. Examples of subjects include, but are not limited to, animals of commercial interest such as birds (hens, ostriches, chicks, geese, partridges, etc.), rabbits, hares, pets (dogs, cats, etc.), sheep, goat cattle (goats, etc.), swine (boars, pigs, etc.), equine livestock (horses, ponies, etc.), cattle (bulls, cows, oxen, etc.); animals of hunting interest, such as stags, deer, reindeer, etc.; and humans. However, in a particular embodiment, the subject is a mammal, particularly the mammal is a human being of any race, sex or age.

In the present invention the term "prevention" means to avoid occurrence of the disease or pathological condition in an individual, particularly when the individual has predisposition for the pathological condition but has not yet been diagnosed. In a preferred embodiment of the present invention, the disease or pathological condition is "alopecia" as further defined herein above.

In the present invention, the term "treat" or "treatment" comprises inhibiting the disease or pathological condition, i.e., stopping its development; relieving the disease or pathological condition, i.e., causing regression of the disease or pathological condition; relieving or reducing at least one of the symptoms or signs of the disease or pathological condition; and/or stabilizing the disease or pathological condition in an individual. In a preferred embodiment of the present invention, the disease or pathological condition is "alopecia" as further defined herein above.

Furthermore, the present invention, also contemplates cellular components, metabolites and molecules secreted by the *Lactobacillus,* preferably *L.rhamnosus,* most preferred *L.rhamnosus* CECT 30580 and *Bifidobacterium,* preferably *B.longum,* most preferred *B.longum* CECT 30616 strains as well as compositions comprising said components and uses thereof for the treatment and/or prevention of alopecia as described herein above. The cellular components of bacteria could include components of the cell wall (such as, but not limited to, peptidoglycan), nucleic acids, membrane components and other, such as proteins, lipids and carbohydrates and combinations thereof (such as lipoproteins, glycolipids or glycoproteins). Metabolites include any molecule produced or modified by the bacterium or microalgae as a result of its metabolic activity during growth, its use in technological processes or during storage of the product (first probiotic composition of the invention). Examples of these metabolites include, but are not limited to, organic and inorganic acids, proteins, peptides, amino acids, enzymes, lipids, carbohydrates, lipoproteins, glycolipids, glycoproteins, vitamins, salts, minerals or nucleic acids. Secreted molecules include any molecule secreted or released to the outside by the bacterium during growth, its use in technological processes (for example, food processing or drugs) or during storage of the product. Examples of these molecules include, but are not limited to, organic and inorganic acids, proteins, peptides, amino acids, enzymes, lipids, carbohydrates, lipoproteins, glycolipids, glycoproteins, vitamins, salts, minerals or nucleic acids.

As understood by those skilled in the art, any of the probiotic compositions of the invention may be formulated for pharmaceutical administration, i.e., forming part of pharmaceutical products to be administered to the subject (either orally, topically, etc., preferably orally), and/or for food administration, i.e. forming part of the foods consumed in the subject's diet, thus being administered orally.

The pharmaceutical compositions may further comprise one or more components which, upon administration to a subject, may further increase, enhance and/or promote the activity of the strains included in any of the probiotic compositions of the invention. As understood by one skilled in the art, the additional components or compounds must be compatible with the strains of any of the probiotic compositions of the invention.

In the context of the present invention, the term "pharmaceutical composition" also encompasses veterinary compositions.

As understood by one skilled in the art, the microorganisms *Lactobacillus,* preferably *L.rhamnosus,* most preferred *L.rhamnosus* CECT 30580 and *Bifidobacterium,* preferably *B.longum,* most preferred *B.longum* CECT 30616 have to be present in the probiotic, pharmaceutical or nutritional compositions of the present invention (as reflected in any aspect or embodiment described throughout the present specification) in an effective amount, particularly in a therapeutically effective amount, so that they can exert their effect of easing, reducing, treating and/or preventing any types of alopecia as further described herein.

In the present invention *"effective amount"* or *"therapeutically effective amount"* is that amount of the component or compound of the probiotic, pharmaceutical or nutritional compositions of the present invention, which when administered to a subject, is sufficient to produce the desired effect. Said component or compound of the probiotic, pharmaceutical or nutritional compositions of the present invention (as reflected in any aspect or embodiment described throughout the present specification), refers to the microorganisms *Lactobacillus,* preferably *L.rhamnosus,* most preferred *L.rhamnosus* CECT 30580 and *Bifidobacterium,* preferably *B.longum,* most preferred *B.longum* CECT 30616. The therapeutically effective amount will vary depending on, for example, age, body weight, general health, sex and diet of the subject, as well as according to the mode and time of administration, excretion rate or drug combination, among other factors.

In yet a further embodiment of the present invention the probiotic pharmaceutical or nutritional compositions of the present invention further comprise at least one bulking agent and/or at least one anti-caking agent and/or at least one coating agent and optionally at least one opacifier.

Preferably said at least one bulking agent of present invention is maltodextrin and/or said at least one coating agent of present invention is gelatin and/or said at least one opacifier is titanium dioxide.

The at least one anti-caking agent of present invention can be selected from the group consisting of magnesium stearate, calcium stearate, silica, silicates, such as sodium or calcium silicate, talc, flour, starch, or combinations thereof.

In a preferred embodiment the bulking agent is maltodextrin and/or cellulose, the anti-caking agent is magnesium stearate and/or silicon dioxide and the coating agent is gelatin or hydroxypropylmethylcellulose.

Specifically, any of the probiotic compositions, probiotic pharmaceutical or nutritional compositions of the present invention (as reflected in any aspect or embodiment described throughout the present specification), are used in a method of treating or preventing alopecia, in particular of non-scarring alopecia, more particularly in a method of easing, reducing, treating and/or preventing alopecia aerate (AA), in a subject or individual, comprising, particularly orally, administering to said subject or individual, an effective amount of the composition thereby treating or preventing said alopecia or AA.

As already indicated previously, it is noted that orally suitable liquid preparations to be used for the compositions of the present invention, may be in the form of, for example, aqueous or oily suspensions, solutions, emulsions, syrups or elixirs, or may be presented as a dry product, such as a lyophilized (freeze-dried) or air-dried product, for constitution with water or other suitable liquid vehicles before use. Such liquid preparations may contain conventional additives such as suspending agents, emulsifying agents, non-aqueous vehicles (which may include edible oils), or preservatives. In one further embodiment, the method of the invention for treating acne in a subject, may further comprise the step of sequentially, subsequently or simultaneously administering to said subject an effective amount of an additional agent, such as isotrenitoin, salicylic acid, witch hazel or Benzoyl peroxide, topical or oral retinoid, spironolactone, an oral contraceptive, azaleic acid, glycolic acid, topical or oral antibiotics, sulfa-based anti-biotics, spf/sunblock, moisturizers or a combination thereof in other embodiments. A person skilled in the art will readily recognize that the components of any treatment composition may be adjusted and optimized based on the symptoms exhibited by the subject, their severity and potential synergistic or antagonistic interactions among the components of such treatment, without exceeding the scope of the invention.

In one further embodiment, the methods and compositions of the invention are used as a supplementary or adjuvant treatment for alopecia, wherein an effective amount of any of the compositions of the present invention, as defined herein, are typically administered orally, either singly or in combination (simultaneously, sequentially or subsequently) with another compound or compounds such isotrenitoin, salicylic acid, witch hazel or Benzoyl peroxide, topical or oral retinoid, spironolactone, an oral contraceptive, azaleic acid, glycolic acid, topical or oral antibiotics, sulfa-based antibiotics, spf/sunblock, or moisturizers. In particular, with antibiotics such as but not limited to topical or oral antibiotics, sulfa-based antibiotics; or hormonal agents for the treatment of acne such as oral contraceptives or antiandrogens. The administration can be carried out in one embodiment, in single unit dosage form with continuous therapy or in another embodiment, in single dose therapy ad libitum. Other embodiments of administration are effective for treating the acne conditions. In other embodiments, any of the compositions of the invention are used when relief of symptoms is specifically required, or, in one embodiment, imminent. Lastly, any of the compositions of the invention may be further used in another embodiment, as a continuous or prophylactic treatment.

Throughout the description and claims the word "comprise" and its variants are not intended to exclude other technical characteristics, additives, components or steps. For those skilled in the art, other objects, advantages and characteristics of the invention will become apparent in part from the description and partly from the practice of the invention. The following examples and figures are provided by way of illustration and are not intended to limit the scope of the present invention.

## EXAMPLES

### EXAMPLE 1:

### 1. GENERAL STUDY DESIGN

This clinical study was designed as a randomized, double-blind, with parallel groups, and placebo controlled with a duration of 24 weeks duration. The 24 weeks of intervention were structured in 6 face-to-face visits in consultation: visit 1 or initial (week 0), visit 2 (week 4), visit 3 (week 8), visit 4 (week 12), visit 5 (week 16) and visit 6 or end of study (week 24). The below table shows the actions carried out in each of the visits that made up the study schematically:

| Visit | V1 | V2 | V3 | V4 | V5 | V6 |
|---|---|---|---|---|---|---|
| Week | 0 | 4 | 8 | 12 | 16 | 24 |
| Information sheet | X | | | | | |
| Informed consent signature | X | | | | | |
| Inclusion and exclusion criteria | X | | | | | |
| Randomization | X | | | | | |
| Patient medical history | X | | | | | |
| Treatment delivery | X | X | X | X | X | X |
| Corticosteroid infiltration | X | X | X | X | X | X |
| Thoracoscopy | X | | | | X | X |
| Plaques counting | X | | | | X | X |
| Patient Photographs | X | X | X | X | X | X |
| DLQI subjective scale | X | | | | | X |
| Blood draw | X | | | | | X |
| Skin and stool sample collection | X | | | | | X |
| Adverse events | | X | X | X | X | X |
| Adherence to treatment | | X | X | X | X | X |

Visit 1 or initial (Week 0)

To recruit patients, all those voluntaries who attended the participating center showing interest in the study with AA diagnosis meeting the criteria indicated in the study protocol were thoroughly informed of the study as well as of the implications of their participation. Before signing the informed consent, the patients had enough time to read and consider the detailed information. They had also the chance to ask any question to the researcher. The patient recruitment process continued until the expected sample size was reached.

Once the informed consent was signed a physician trained for the study conducted the initial interview where it was verified that the patient met all the inclusion criteria and none of the exclusion criteria. The researcher proceeded to assign the patient a participant number in the study. According to a previously prepared randomization list, the patient was assigned the treatment received during the study by contacting the randomization centre. The randomized list was kept by a person in charge from outside the study, whom the investigator contacted each time a new patient had to be included.

Likewise, in this initial visit, the clinical history and capillary measurements were made: trichoscopy, plaques counting and photographs. In addition, the patient underwent a blood draw, and a skin sample from the area of plaques as well as sample of faeces was collected. Finally, following the therapeutic protocol, a local infiltration of corticosteroids was performed and data from the DLQI subjective scale was collected. The patient was given enough treatment to cover the period until the next scheduled visit according to its assigned code.

Intermediate visits 2, 3 and 4 (Weeks 4, 8 and 12 respectively)

In addition to assessing the symptoms of the disease, the infiltration of topical corticosteroids and taking photographs was performed during these visits. The researcher recorded the adverse events reported by the patient (including the start date and in case those adverse events had already been resolved at the time of the visit the end date) as well as the accounting of the remaining product to evaluate treatment compliance in the Case Report Form (CRF). The patient was given enough treatment to cover the period until the next scheduled visit according to its assigned code.

Intermediate Visit 5 (Week 16)

Besides performing the same procedures as in previous intermediate visits 2, 3 and 4, in this visit capillary measurements were added. The patient was given enough treatment to cover the period until the next scheduled visit according to its assigned code.

Visit 6 or final (Week 24)

During this final visit, the same procedures as in visit 1 were conducted, including capillary measurements and sample collection. Possible adverse events were recorded, specifying the start and end dates, and the treatment received, if any. In addition, patient's leftover study product was collected.

1.1 PARTICIPANTS SELECTION CRITERIA

a) Inclusion criteria

- AA diagnosis by clinical criteria

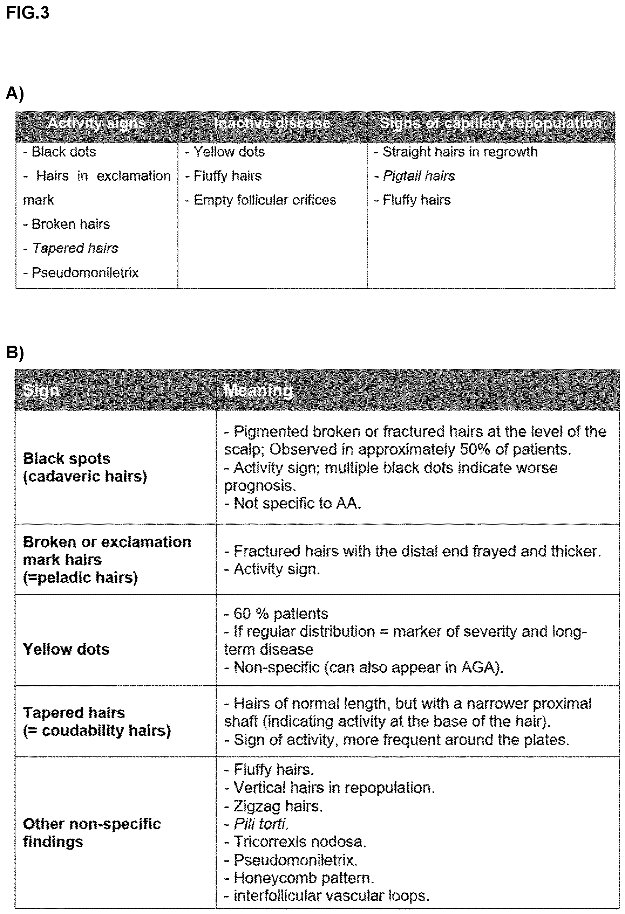

- Show at least 2 signs of AA activity visualized by trichoscopy and defined in Fig.3

b) Exclusion criteria

- Allergies or contraindication to take any of the components of the product under study

- Topical or systemic use of antifungals and antibiotics in the previous 2 weeks

- Consumption of probiotics in the previous 2 months

- Participation in clinical studies in the previous 2 months

- Pregnancy and/or lactation

1.2 TREATMENT

a) Description, Ingredients and Composition

The product under study was a probiotic in capsule format administered orally containing probiotic strains at concentrations equal to or greater than $1 \times 10^9$ cfu/dose. This probiotic product contains a mixture of strains of *Lactobacillus* and *Bifidobacterium,* specifically the *L.rhamnosus* CECT 30580 concentration is 50% and the B. *longum* CECT 30616 concentration is 50% with respect to the total concentration of microorganisms present in the composition.

The placebo used contains only maltodextrin, masked in an identical and indistinguishable format to that of the probiotic.

b) Applications

The hypothesis was formulated that dietary supplementation of probiotics, with functional capacities on mucocutaneous tissue and its adnexa, could benefit patients with AA, via improvements in the microbiome profiles of patients.

c) Storage

The product must be stored in a cool and dry place at room temperature, always below 25°C

d) Dose and Usage

The daily product dose is 1 oral capsule a day. The capsules are of adequate size to be easily swallowed. The capsules can be opened to disperse the content in a glass with a little water. Avoid taking the capsules with hot drinks.

e) Adverse Effects

No significant adverse effects have been recorded after the consumption of probiotics. Previous studies have described mild digestive discomfort in the first days of taking the product to study.

1.3 VARIABLES

a) Main variable

- Presence or not of signs of capillary repopulation, signs of inactivity and/or signs of AA activity, visualized by trichoscopy and defined in Fig.3.

b) Secondary variables

- AA plaques number

- AA category/type: "single", "multifocal", "total" or "universal"

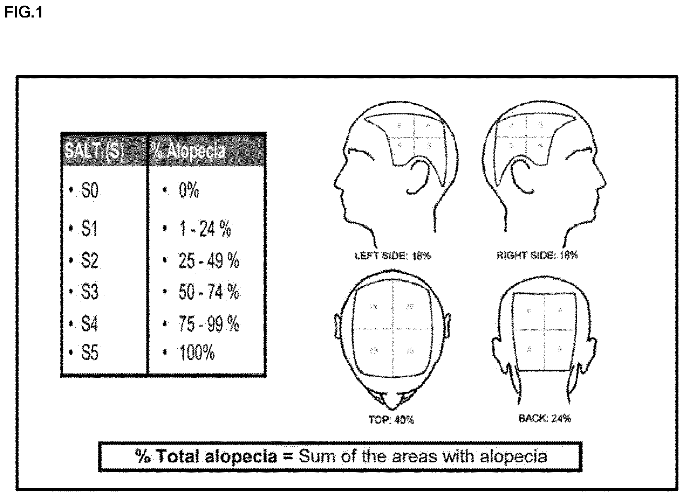

- Affected skin surface according to the SALT scale (Fig.1) (only for plaques on the scalp)

- Haemoglobin, leukocytes, platelets, urea, creatinine, TSH, C-reactive protein, AST, ALT, IL-10, tumor necrosis factor (TNF), IgE values

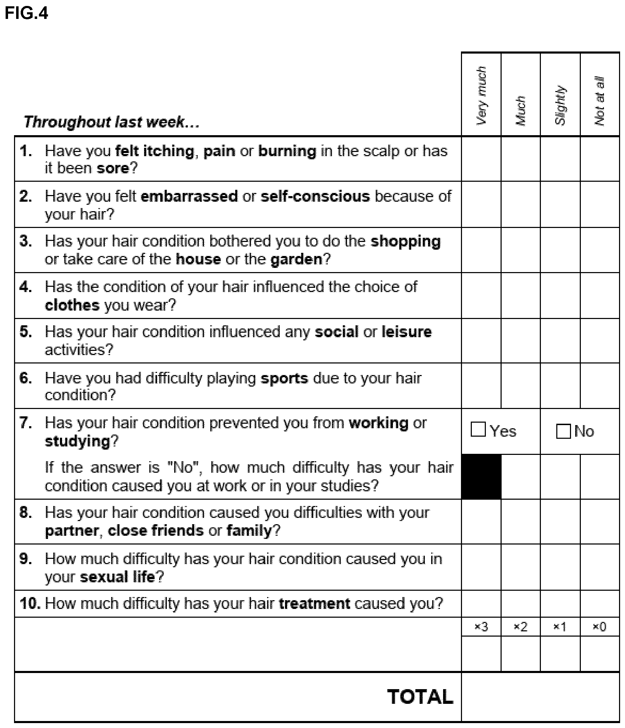

- Score on the subjective Dermatology Life Quality Index (DLQI) scale (Fig.4)

- Composition of the microbiota, by sequencing the R16s gene, from a skin sample and a stool sample

- Adherence to treatment

c) Security variable

- Number, type and severity of all adverse effects that occur during the study, related or not to the intake of the probiotic product

1.4 DETERMINATION OF SAMPLE SIZE

Twenty-six patient volunteers participated in the study, proportionately divided 1:1 into the placebo and probiotic groups, respectively. A sequential non-probabilistic sampling was used in which the first 26 patients who attended the consultation and who met the inclusion criteria and none of the exclusion criteria above described were selected.
A monitoring loss rate of 10% was estimated during the study design. The final data analysis included all the patients that finalized the visits programmed for the study (evaluable subjects), while all other patients were excluded from the analysis, not being replaced by new patients after being removed from the study.

1.5 RANDOMIZATION METHOD

Double blind randomized clinical study, for which the treatment was randomly assigned by chance. Not even the patients or the researchers involved in the study knew the assigned treatment, because these were previously coded. The treatment boxes were labelled with those numbers and did not show distinctive signs of the treatment group. The patients on the list were randomized to each of the two intervention groups in a 1:1 ratio (50% probability in both groups) in each stratum. These lists were kept by the head of the randomization center and were hidden from the study staff and participants.

1.6 DEMOGRAPHIC DATA, BASELINE CHARACTERISTICS AND STUDY DATA

Baseline demographic data collected at the start of the clinical study were used to describe the population under study and to assess homogeneity between the two follow-up groups after randomization.

1.7 EFFICACY ANALYSIS

The evolution of the disease was evaluated throughout a period of 24 weeks. To this end a medical examination and clinical history was performed by the researchers in the initial interview. Clinical data related to: activity and inactivity of the disease, capillary repopulation, number of alopecia areata plaques, affected skin surface, analytical parameters, microbiota samples and impact scale on the patient's quality of life was also collected in this initial interview. At the end of the follow-up period and during the final interview, these data were collected again to obtain a comparison and observe possible changes.
To study the main variable, capillary trichoscopy was performed at the initial visit, at intermediate follow-up visit (week 16), and at the final visit (week 24). This way, it was possible to evaluate the differences and evolution during the follow-up period at week and at the end of the study.
To study the secondary variables the following was collected:

- Plaques count at: initial visit, intermediate follow-up visit (week 16), and final visit (week 24)

- Patients scalp photographs at: initial visit, all intermediate follow-ups visits, and final visit

- SALT scale to assess the affected skin surface at: initial visit, intermediate follow-up visit (week 16), and final visit (week 24)

- Blood analysis at initial and final visit

- Subjective Dermatology Life Quality Index scale at initial and final visit

- Skin and stool samples at initial and final visit

- Capsules left over in all intermediate visits and in final visit to assess the adherence to treatment

In this study, the data of the subjects was collected in a Case Report Form (CRF), and then introduced into a database generated with the IBM SPSS Statistics version 22.0. statistics program. The quantitative variables are analysed using the statistical sample U of Mann-Whitney or t of Student, while the qualitative variables were analysed using contingency tables.

2. RESULTS

2.1 POPULATION CARACTERISTCS

26 patients were evaluated in the study. The distribution of the sample was left with 13 patients in the placebo group versus 13 patients in the probiotic group.

The below, tables 1 and 2 show the general characteristics of the population included in the study: demographic data, analytical data and clinical variables associated with the study.

Table 1: Demographic and clinical data of the patients included in the study

| | |
|---|---|
| **Gender (Woman),** N (%) | 18 (69.20%) |
| **Age (years),** Average $\pm$ SD | 39.50 $\pm$ 12.59 |
| **Weight (kg),** Average $\pm$ SD | 71.62 $\pm$ 18.76 |
| **Height (cm),** Average $\pm$ SD | 169.00 $\pm$ 7.80 |
| **Smoker,** N (%) | 11 (42.31%) |
| **Allergies and intolerances,** N (%) | 5 (19.23%) |
| **Previous pathologies** | 12 (46.15%) |
| Endocrine, N (%) | 4(15.38%) |
| Rheumatologic, N (%) | 1 (3.85%) |
| Metabolic, N (%) | 1 (3.85%) |
| Gynecological, N (%) | 1 (3.85%) |
| Skin, N (%) | 3(11.54%) |
| Digestive, N (%) | 1 (3.85%) |
| Other, N (%) | 2 (7.69%) |
| **Trichoscopy** | |
| **Activity sign $\times$ number of plaques,** Average $\pm$ SD | 10.31 $\pm$ 6.80 |
| **Inactivity Signs/number of plaques,** Average $\pm$ SD | 0.56 $\pm$ 0.46 |
| **Signs of capillary repopulation/number of plaques,** Average $\pm$ SD | 0.73 $\pm$ 0.52 |
| **Number of alopecia plaques,** Average $\pm$ SD | 3.62 $\pm$ 0.43 |
| **Alopecia type** | |
| Multifocal, N (%) | 20 (76.92%) |
| Single, N (%) | 5 (19.23%) |
| Universal, N (%) | 1 (3.85%) |
| **SALT scale** | |
| S1, N (%) | 14 (53.85%) |
| S2, N (%) | 11 (42.31%) |
| S3, N (%) | 0 |
| S4, N (%) | 1 (3.85%) |
| **DLQI scale,** Average $\pm$ SD | 4.12 $\pm$ 4.42 |

Table 2: Haematology and blood biochemistry data, descriptive of the patients included in the study

| | |
|---|---|
| **Leukocytes** (*10^3/$\mu$L), Average $\pm$ SD | 8.40 $\pm$ 1.44 |

(continued)

| | | |
|---|---|---|
| | Median [min-max] | 8.08 [5.58 - 11.98] |
| **Hemoglobin** (g/dL), | | |
| | Average ± SD | 14.06 ± 1.22 |
| | Median [min-max] | 13.75 [12.10 - 17.10] |
| **Platelets** (*10^3/$\mu$L), | | |
| | Average ± SD | 281.92 ± 60.53 |
| | Median [min-max] | 270.5 [198 - 457] |
| **ALT** (U/L), | | |
| | Average ± SD | 18.08 ± 15.11 |
| | Median [min-max] | 14.5 [5 - 82] |
| **AST** (U/L), | | |
| | Average ± SD | 41.96 ± 20.65 |
| | Median [min-max] | 36 [16 - 98] |
| **Urea** (mg/dL), | | |
| | Average ± SD | 35.50 ± 8.81 |
| | Median [min-max] | 35 [16 - 51] |
| **Creatinine** (mg/dL), | | |
| | Average ± SD | 0.76 ± 0.22 |
| | Median [min-max] | 0.74 [0.42 - 1.16] |
| **TSH** ($\mu$U/mL), | | |
| | Average ± SD | 1.20 ± 0.77 |
| | Median [min-max] | 1.15 [0.01 -2.77] |
| **CRP** (mg/dL), | | |
| | Average ± SD | 0.54 ± 0.14 |
| | Median [min-max] | 0.49 [0.49 -1] |
| **IL-10** (pg/mL), | | |
| | Average ± SD | 2.67 ± 5.49 |
| | Median [min-max] | 1.50 [1.50 - 29.5] |
| **TNF** (pg/mL), | | |
| | Average ± SD | 4.27 ± 0.66 |
| | Median [min-max] | 3.90 [3.90 - 6.60] |
| **IgE** (KU/L), | | |
| | Average ± SD | 94.79 ± 133.79 |
| | Median [min-max] | 39.55 [1.90 - 508.10] |

## 2.2 DESCRIPTION BY GROUPS: HOMOGENEITY

To clarify whether the patient randomization process had been effective, a homogeneity analysis of the descriptive and analytical variables of the population was performed for both groups: probiotic and placebo (Tables 3 and 4).

Table 3: Homogeneity analysis of demographic and clinical data in baseline visit

| | PLACEBO (N=13) | PROBIOTIC (N=13) |
|---|---|---|
| **Gender (Woman),** N (%) | 11 (84.61 %) | 7 (53.85%) |
| **Age (years),** Media ± SD | 43.46 ± 14.49 | 35.54 ± 9.27 |
| **Weight (kg),** Media ± SD | 69.38 ± 17.04 | 73.85 ± 20.80 |
| **Height (cm),** Media ± SD | 166.62 ± 6.33 | 171.38 ± 8.63 |

(continued)

|  | PLACEBO (N=13) | PROBIOTIC (N=13) |
|---|---|---|
| **Smoker,** N (%) | 5 (35.46%) | 6 (46.15%) |
| **Allergies and intolerances,** N (%) | 3 (23.08%) | 2 (15.38%) |
| **Previous pathologies** | 6 (46.15%) | 6 (46.15%) |
| Endocrine, N (%) | 3 (23.08%) | 1 (7.69%) |
| Rheumatologic, N (%) | 0 | 1 (7.69%) |
| Metabolic, N (%) | 1 (7.69%) | 0 |
| Gynecological, N (%) | 1 (7.69%) | 0 |
| Skin, N (%) | 1 (7.69%) | 2 (15.38%) |
| Digestive, N (%) | 0 | 1 (7.69%) |
| Other, N (%) | 0 | 1 (7.69%) |
| **Trichoscopy** | | |
| **Activity sign × number of plaques,** Average ± SD | 10.38 ± 7.56 | 10.23 ± 6.27 |
| **Inactivitysigns / number of plaques**, Average ± SD | 0.72 ± 0.57 | 0.41 ± 0.26 |
| **Signs of capillary repopulation / number of plaques,** Average ± SD | 0.78 ± 0.59 | 0.68 ± 0.46 |
| **Number of alopecia plaques,** Average ± SD | 3.31 ± 2.43 | 3.92 ± 1.93 |
| **Alopecia type** | | |
| Multifocal, N (%) | 8(61.54%) | 12(92.31%) |
| Unique, N (%) | 4 (30.77%) | 1 (7.69%) |
| Universal, N (%) | 1 (7.69%) | 0 |
| **SALT scale** | | |
| S1, N (%) | 8 (61.54%) | 6 (46.15%) |
| S2, N (%) | 4 (30.77%) | 7 (53.85%) |
| S3, N (%) | 0 | 0 |
| S4, N (%) | 1 (7.69%) | 0 |
| **DLQI scale,** Media ± SD | 4.31 ± 4.63 | 3.92 ± 4.39 |

Table 4: Homogeneity analysis of blood test data in baseline visit

|  | PLACEBO (N=13) | PROBIOTIC (N=13) |
|---|---|---|
| **Leukocytes** (*10^3/μL), | | |
| Average ± SD | 8.29 ± 1.35 | 8.51 ± 1.57 |
| Median [min-max] | 8.22 [6.25 - 10.54] | 7.94 [ 5.58 - 11.98] |
| **Hemoglobin** (g/dL), | | |
| Average ± SD | 13.91 ± 1.21 | 14.22 ± 1.27 |
| Median [min-max] | 13.50 [12.10 - 16.40] | 14.30 [12.50 - 17.10] |
| **Platelets** (*10^3/μL), | | |
| Average ± SD | 290.46 ± 54.61 | 273.38 ± 67.03 |
| Median [min-max] | 271 [224 - 377] | 270 [198 - 457] |
| **ALT** (U/L), | | |

(continued)

|  | | PLACEBO (N=13) | PROBIOTIC (N=13) |
|---|---|---|---|
|  | Average ± SD | 13.31 ± 5.27 | 22.85 ± 19.97 |
|  | Median [min-max] | 13 [6 - 28] | 18 [5 - 82] |
| **AST** (U/L), | | | |
|  | Average ± SD | 42.23 ± 19.74 | 41.69 ± 22.32 |
|  | Median [min-max] | 39 [16 - 73] | 32 [19 - 98] |
| **Urea** (mg/dL), | | | |
|  | Average ± SD | 38.54 ± 6.91 | 32.46 ± 9.68 |
|  | Median [min-max] | 42 [24 - 49] | 31 [16 - 51] |
| **Creatinine** (mg/dL), | | | |
|  | Average ± SD | 0.77 ± 0.21 | 0.75 ± 0.23 |
|  | Median [min-max] | 0.75 [0.47 - 1.16] | 0.74 [0.42 - 1.16] |
| **TSH** ($\mu$U/mL), | | | |
|  | Average ± SD | 1.12 ± 0.77 | 1.27 ± 0.79 |
|  | edian [min-max] | 1.13 [0.04 - 2.17] | 1.17 [0.01 - 2.77] |
| **CRP** (mg/dL), | | | |
|  | Average ± SD | 0.51 ± 0.06 | 0.57 ± 0.19 |
|  | Median [min-max] | 0.49 [0.49 - 0.70] | 0.49 [0.49 - 1] |
| **IL-10** (pg/mL), | | | |
|  | Average ± SD | 1.70 ± 0.69 | 3.65 ± 7.77 |
|  | Median [min-max] | 1.50 [1.50 - 4] | 1.50 [1.50 - 29.50] |
| **TNF** (pg/mL), | | | |
|  | Average ± SD | 4.35 ± 0.83 | 4.19 ± 0.46 |
|  | Median [min-max] | 3.90 [3.90 - 6.60] | 3.90 [3.90 - 5.40] |
| **IgE** (KU/L), | | | |
|  | Average ± SD | 49.35 ± 63.49 | 140.23 ± 169.66 |
|  | Median [min-max] | 28.60 [1.90 - 230] | 54.90 [5.10 - 508] |

2.3 CONSORT DIAGRAM

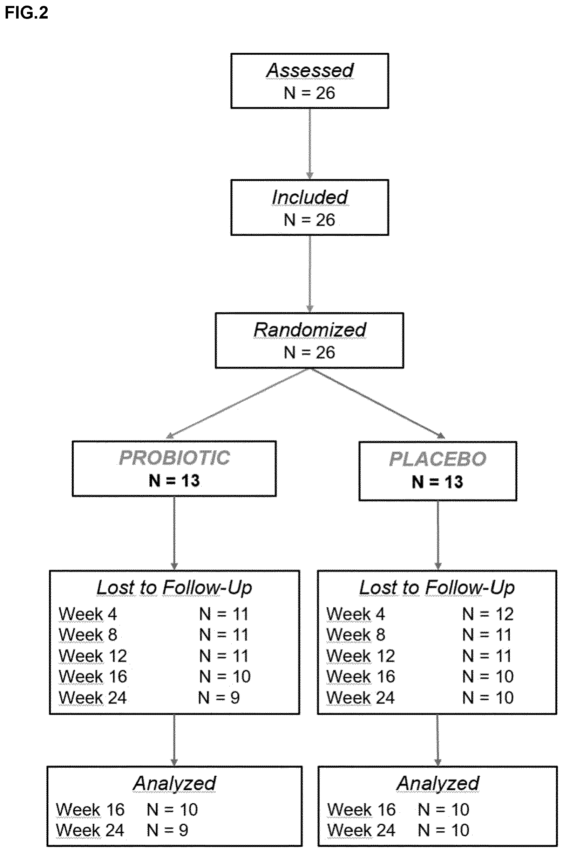

As can be seen in the consort diagram of Fig.2, during the development of the study, 26 patients were assessed. All of them were included in the study, randomized, and assigned to one of the follow-up groups. The patients were distributed in such a way that there were 13 patients in the placebo group and 13 in the probiotic group. 7 patients (4 in the probiotic group and 3 in the placebo group) decided to drop out voluntarily at some point of the study. The total number of patients present at each of the 6 visits that comprised the study was 19.

The analysis of the results has been carried out by intention to treat, with all the available data of the patients included, that is, all the patients for whom we have comparative follow-up data have been considered. Comparative data was not available for 6 of the 26 patients included. There were at least 2 comparative visits in 20 patients.

2.4 EFFICIENCY ANALYSIS

Presence or not of signs of capillary repopulation, signs of inactivity and/or signs of AA activity, visualized by trichoscopy and defined in Fig.3.

The score ranges for each of the 3 variables based on the signs observed by trichoscopy in the set of plates were:

-    Activity: From 0 to 5 points.

-    Inactivity: From 0 to 3 points.

-    Repopulation: From 0 to 3 points.

For the results interpretation, a score was calculated based on the number of plaques and the number of signs of activity, inactivity and repopulation present.

Resolved plaques are given the maximum upgrade value in each of the variables:

- Activity: + 5 points

- Inactivity: + 3 points

- Repopulation: + 3 points

To assess the activity variable at each visit, the following formula was used:

Number of signs of activity x number of alopecia plaques

To assess the difference of the activity variable between visits, the following formula was used:

*(Number of signs of activity x number of alopecia plaques) – (number of resolved plaques x maximum value of improvement in activity)*

To assess the inactivity and repopulation variables score at each visit the following formula was used:

*Number of signs of inactivity / number of alopecia plaques*

*Number of signs of repopulation / number of alopecia plaques*

To assess the difference between visits of the inactivity and repopulation variables, the following formula was used:

*(Number of signs of inactivity / number of alopecia plaques) + (number of resolved plaques x maximum value of improvement in inactivity)*

*(Number of signs of repopulation / number of alopecia plaques) + (number of resolved plaques x maximum value of improvement in repopulation)*

**[0142]** * To be taken into account that the activity variable is inversely proportional to the inactivity and repopulation variables.

The following table (Table 5) shows the evolution by number of patients during the study with respect to these 3 variables.

Table 5: Changes per patient in signs of activity, inactivity and repopulation in capillary trichoscopy

| Variable | Range | N | Group | Patients who worsen N (%) | Patients without changes N (%) | Patients who improve N (%) | P |
|---|---|---|---|---|---|---|---|
| Activity | V1 to V5 | 10 | Placebo | 5 (50%) | 1 (10%) | 4 (40%) | 0.470 |
| | | 10 | Probiotic | 4 (40%) | 0 | 6 (60%) | |
| | V1 to V6 | 10 | Placebo | 3 (30%) | 2 (20%) | 5 (50%) | 0.351 |
| | | 9 | Probiotic | 4 (44.44%) | 0 | 5 (55.55%) | |
| Inactivity | V1 to V5 | 10 | Placebo | 4 (40%) | 3 (30%) | 3 (30%) | 0.472 |
| | | 10 | Probiotic | 4 (40%) | 1 (10%) | 5 (50%) | |
| | V1 to V6 | 10 | Placebo | 3 (30%) | 3 (30%) | 4 (40%) | 0.187 |
| | | 9 | Probiotic | 3 (33.33%) | 0 | 6 (66.67%) | |
| Repopulation | V1 a V5 | 10 | Placebo | 5 (50%) | 2 (20%) | 3 (30%) | 0.327 |
| | | 10 | Probiotic | 6 (60%) | 0 | 4 (40%) | |
| | V1 to V6 | 10 | Placebo | 4 (40%) | 3 (30%) | 3 (30%) | 0.178 |
| | | 9 | Probiotic | 4 (44.4%) | 0 | 5 (55.5%) | |

The following table (Table 6), in the analysis of these 3 variables, shows the differences by group in the mean score.

Table 6: Differences in signs of activity, inactivity and repopulation

| Variable | Range | N | Group | Difference Average ± SD | P |
|---|---|---|---|---|---|
| Activity | V1 to V5 | 10 | Placebo | + 1.20 ± 12.25 | 0.991 |
| | | 10 | Probiotic | + 1.10 ± 24.23 | |
| | V1 to V6 | 10 | Placebo | - 3.00 ± 7.82 | 0.670 |
| | | 9 | Probiotic | - 5.78 ± 17.51 | |
| Inactivity | V1 to V5 | 10 | Placebo | + 0.87 ± 2.08 | 0.189 |
| | | 10 | Probiotic | + 3.32 ± 5.14 | |
| | V1 to V6 | 10 | Placebo | + 2.26 ± 4.25 | 0.313 |
| | | 9 | Probiotic | + 4.58 ± 5.48 | |
| Repopulation | V1 to V5 | 10 | Placebo | + 0.93 ± 2.11 | 0.218 |
| | | 10 | Probiotic | + 3.32 ± 5.38 | |
| | V1 to V6 | 10 | Placebo | + 2.02 ± 4.25 | 0.308 |
| | | 9 | Probiotic | + 4.40 ± 5.56 | |

2.5 SECONDARY ANALYSIS

2.5.1 Number of AA plaques

The following table (Table 7) shows the evolution by patients in the number of AA plaques during the follow-up period.

Table 7: Changes per patient in the number of AA plaques

| Variable | Range | N | Group | Patients who worsen N (%) | Patients without changes N (%) | Patients who improve N (%) | P |
|---|---|---|---|---|---|---|---|
| Number of plaques | V1 to V5 | 10 | Placebo | 5 (50%) | 3 (30%) | 2 (20%) | 0.302 |
| | | 10 | Probiotic | 4 (40%) | 1 (10%) | 5 (50%) | |
| | V1 to V6 | 10 | Placebo | 3 (30%) | 4 (40%) | 3 (30%) | 0.100 |
| | | 9 | Probiotic | 4 (44.44%) | 0 | 5 (55.55%) | |

Of the 8 patients who improved the number of alopecia plaques at the end of the study, 2 of them resolved all the plaques. These 2 patients belonged to the probiotic group.

The following table (Table 8) shows the mean difference in the number of plaques during the follow-up period.

Table 8: Differences in the number of plaques

| Variable | Range | N | Group | Difference Average $\pm$ SD | P |
|---|---|---|---|---|---|
| Number of plaques | V1 to V5 | 10 | Placebo | + 0.70 $\pm$ 1.89 | 0.951 |
| | | 10 | Probiotic | + 0.80 $\pm$ 4.71 | |
| | V1 to V6 | 10 | Placebo | - 0.1 $\pm$ 1.97 | 0.925 |
| | | 9 | Probiotic | - 0.22 $\pm$ 3.31 | |

2.5.2 AA category/type: "single", "multifocal", "total" and "universal"

The following table (Table 9) shows the evolution by number of patients in the type of AA during the follow-up period.

Table 9: AA category

| Variable | Range | N | Group | Patients who worsen N (%) | Patients without changes N (%) | Patients who improve N (%) | P |
|---|---|---|---|---|---|---|---|
| Alopecia type | V1 to V5 | 10 | Placebo | 0 | 9 (90%) | 1 (10%) | 1.000 |
| | | 10 | Probiotic | 0 | 9 (90%) | 1 (10%) | |
| | V1 to V6 | 10 | Placebo | 0 | 9 (90%) | 1 (10%) | 0.453 |
| | | 9 | Probiotic | 0 | 7 (77.8%) | 2 (22.2%) | |

2.5.3 Affected skin surface according to the SALT scale (Annex II) (only for plaques on the scalp)

The following table (Table 10) shows the evolution by number of patients with respect to the SALT scale that evaluates the affected skin surface.

Table 10: Changes per patient in SALT scale

| Variable | Range | N | Group | Patients who worsen N (%) | Patients without changes N (%) | Patients who improve N (%) | P |
|---|---|---|---|---|---|---|---|
| SALT Scale | V1 to V5 | 10 | Placebo | 1 (10%) | 8 (80%) | 1 (10%) | 0.513 |
| | | 10 | Probiotic | 2 (20%) | 8 (80%) | 0 | |
| | V1 to V6 | 10 | Placebo | 2 (20%) | 6 (60%) | 2 (20%) | 0.509 |
| | | 9 | Probiotic | 1 (11.11%) | 4 (44.44%) | 4 (44.44%) | |

2.5.4 Hemoglobin, leukocytes, platelets, urea, creatinine, TSH, C-reactive protein, AST, ALT, IL-10, tumor necrosis factor (TNF), IgE values

The below table (Table 11) shows the final visit analytical data.

Table 11: Final visit analytical data

| | PLACEBO (N=13) | PROBIOTIC (N=13) |
|---|---|---|
| **Leukocytes** (*10^3/$\mu$L), | | |
| Average $\pm$ SD | 7.68 $\pm$ 2.18 | 7.81 $\pm$ 2.10 |
| Median [min-max] | 7.33 [4.95 - 12.35] | 7.50 [5.23 - 12.35] |
| **Hemoglobin** (g/dL), | | |
| Average $\pm$ SD | 14.02 $\pm$ 1.71 | 14.11 $\pm$ 0.54 |
| Median [min-max] | 13.80 [12.10 - 17.20] | 14.10 [13.40 - 14.80] |
| **Platelets** (*10^3/$\mu$L), | | |
| Average $\pm$ SD | 262 $\pm$ 50.74 | 268.89 $\pm$ 57.55 |
| Median [min-max] | 232 [212 - 336] | 275 [174 - 379] |
| **ALT** (U/L), | | |
| Average $\pm$ SD | 12.13 $\pm$ 3.52 | 19.00 $\pm$ 9.94 |
| Median [min-max] | 12 [6 - 19] | 17 [7 - 40] |
| **AST** (U/L), | | |
| Average $\pm$ SD | 38.38 $\pm$ 11.87 | 46.38 $\pm$ 21.86 |
| Median [min-max] | 39.5 [19 - 54] | 39.5 [19 - 85] |
| **Urea** (mg/dL), | | |
| Average $\pm$ SD | 28.88 $\pm$ 7.49 | 33.88 $\pm$ 8.18 |
| Median [min-max] | 27 [19 - 39] | 37 [18 - 42] |
| **Creatinine** (mg/dL), | | |
| Average $\pm$ SD | 0.66 $\pm$ 0.12 | 0.90 $\pm$ 0.24 |
| Median [min-max] | 0.63 [0.54 - 0.91] | 0.98 [0.52 - 1.29] |
| **TSH** ($\mu$U/mL), | | |
| Average $\pm$ SD | 1.72 $\pm$ 1.08 | 1.99 $\pm$ 0.92 |
| Median [min-max] | 1.31 [0.34 - 3.48] | 1.87 [0.72 - 4.12] |
| **CRP** (mg/dL), | | |
| Average $\pm$ SD | 0.53 $\pm$ 0.11 | 0.49 $\pm$ 0.00 |
| Median [min-max] | 0.49 [0.49 - 0.80] | 0.49 [0.49 - 0.49] |
| **IL-10** (pg/mL), | | |
| Average $\pm$ SD | 1.50 $\pm$ 0.00 | 7.84 $\pm$ 17.74 |
| Median [min-max] | 1.50 [1.50 - 1.50] | 1.50 [1.50 - 52.20] |
| **TNF** (pg/mL), | | |
| Average $\pm$ SD | 3.90 $\pm$ 0.00 | 3.99 $\pm$ 0.25 |
| Median [min-max] | 3.90 [3.90 - 3.90] | 3.90 [3.90 - 4.60] |
| **IgE** (KU/L), | | |
| Average $\pm$ SD | 49.00 $\pm$ 46.89 | 185.22 $\pm$ 239.36 |
| Median [min-max] | 29.45 [10.2 - 138] | 57.70 [7.5 - 706] |

No significant differences are observed between the analytical data of the final visit and the values of the initial visit.

2.5.5 Score on the subjective Dermatology Life Quality Index (DLQI) scale (Fig.1)

The following table (Table 12) shows the evolution by number of patients with respect to the scores on the DLQI dermatological quality of life scale.

Table 12: Changes per patient in the DLQI scale

| Variable | Range | N | Group | Patients who worsen N (%) | Patients without changes N (%) | Patients who improve N (%) | P |
|---|---|---|---|---|---|---|---|
| DLQI Scale | V1 to V6 | 10 | Placebo | 2 (20%) | 1 (10%) | 7 (70%) | 0.521 |
| | | 9 | Probiotic | 3 (33.3%) | 2 (22.2%) | 4 (44.4%) | |

2.5.6 Composition of the microbiota in skin and feces. See Example 2.

2.5.7 Patient adherence to treatment

The following table (Table 13) shows adherence to treatment in both groups.

Table 13: Adherence to treatment

| Range | N | Group | Compliance Average ± SD |
|---|---|---|---|
| V1 to V6 | 10 | Placebo | 83.77 ± 8.513 |
| | 9 | Probiotic | 82.80 ± 6.331 |

2.5.8 Security Analysis

Throughout the study, the researchers collected all the adverse events that arose during the follow-up period, between visit 1 and visit 6. In total, 5 adverse events were reported: 1 in the probiotic group and 4 in the placebo group. Of the 5 adverse events collected, 3 of them were potentially attributable to the treatment taken by the patients, all of them in the placebo group

Table 14: Description of adverse events potentially attributable to treatment

| PLACEBO (N=13) | PROBIOTIC (N=13) |
|---|---|
| Abdominal pain Constipation Diarrhea | - |

**3. CONCLUSIONS**

1. In the activity variable, 5/9 (55.5%) of the patients in the probiotic group improved between the initial and final visits, while 5/10 (50%) in the placebo group improved (Table 5). The mean score difference in the probiotic group was -5.78 points, versus -3.00 in the placebo group (Table 6).

2. In the capillary repopulation variable, 5/9 (55.5%) of the patients in the probiotic group improved between the initial and final visits, while 3/10 (30%) in the placebo group improved (Table 5). The mean score difference in the probiotic group was +4.40 points, versus +2.02 in the placebo group (Table 6).

3. In the inactivity variable, 6/9 (66.7%) of the patients in the probiotic group improved between the initial and final visit, while in the placebo group 4/10 (40%) improved (Table 5). The mean score difference in the probiotic group was +4.58 points, versus +2.26 in the placebo group (Table 6).

4. In the number of plaques variable, 5/9 (55.5%) of the patients in the probiotic group decreased the number of plaques from start to end, while in the placebo group the improvement was 3/10 (30%) (Table 7). No differences were observed in the mean improvement in the number of plaques per group (Table 8).

5. Regarding the AA category, no relevant differences were observed. Most of the patients in both groups maintained the initial typology at the end of the study (Table 9).

6. In the SALT scale variable of skin surface affected by alopecia, 4/9 (44.4%) of the patients in the probiotic group improved the score between the initial and final visit, while in the placebo group this improvement was 2 /10 (20%) (Table 10).

7. In the analytical values, no significant changes are observed between the initial and final visit (Table 11).

8. In the DLQI dermatological quality of life scale, no significant differences were observed between groups (Table 12).

9. Composition of microbiota. See Example 2

10. Patient adherence to treatment was 82.80% on average in the probiotic group compared to 83.77% in the placebo group. Both values are considered acceptable and no differences between groups are observed (Table 13).

11. The studied product was shown to be safe, with no attributable adverse events reported in the probiotic group (Table 14).

Data from this pilot study showed a beneficial effect of the probiotic preparation with:

a) improvement in signs of activity, inactivity of the disease as well as in capillary repopulation

b) Number of plaques decrease

c) Smaller area of skin affected

The differences between groups throughout the study were clinically significant.

## EXAMPLE 2:

### 1. ANALYSIS OF MICROBIOTA OF STOOL AND SKIN SAMPLES

Stool samples and skin samples were taken of patients included in this study to assess the change in the microbiota of the samples after treatment.

### 2. MATERIAL AND METHODS

Extraction of genetic material (DNA) of the stool samples and skin samples of patients included in this study was performed using a combination of mechanical and enzymatic disruption of cell walls and membranes in order to increase the yield of extraction and not bias the presence of bacteria with cell wall (Gram +). The genetic material obtained by extraction was measured to assess quality and quantity using a Nanodrop 2000 ThermoScientific to inspect ratios 260/280 and 260/230 indicating the extraction quality (presence of PCR inhibitors, pigment, etc.). Later, after verifying the quality of the same, massive sequencing libraries were conducted, capturing the hypervariable region V3-V4 of the bacterial 16s rRNA gene (based on Klindworth A, et al. (2013) Nucleic Acids Res 41: e1) according to the protocol described by Illumina for analysis of the microbial composition based on the capture of 16s rRNA. Each library was quantified with Quant-iT PicoGreen by Invitrogen and mixed equimolarly for subsequent sequencing.

Samples were sequenced in MiSeq platform in a combination of 300 "Paired-End" cycles. The resulting FASTQ files were treated to ensure high quality sequence analysis. For this purpose, a quality control was conducted consisting of:

1. Joining the ends to reconstruct unique sequences using the program 'pear' v0.9.6. (Zhang J, et al (2014) Bioinformatics 30 (5):614-20).

2. Elimination of sequencing adapters and capture primers from the hypervariable regions V3 and V4 with the program cutadapt version 1.9.1. (Martin M (2010) EMBnet.journal [S.L.], 17 (1): 10-12. ISSN 2226-6089.).

3. Elimination of low-quality sequences using FASTX-ToolKit version 0.91.

4. Elimination of chimeras produced by PCR using the UCHIME program (Dec 2015) (Edgar RC, et al (2011) Aug 15; 27 (16): 2194-200) and the last database of chimeras.

The resulting samples were compared between groups (probiotic group vs placebo group). Each of the sequences for which a score was obtained of ninety-five percent (95%) identity was inspected at different taxonomic of levels: Phylum, Family, Genus and Species. The R statistical package was used for statistics, to construct graphs for principal component analysis (PCA).

## 3. RESULTS

### 3.1 GUT MICROBIOTA

*A. Baseline*

In the gut microbiota of the included alopecia areata patients in the clinical trial:

- The phyla with the highest relative abundance were Firmicutes (61.46 $\pm$ 8.56%), Bacteroidetes (25.21 $\pm$ 7.09%) and Actinobacteria (10.32 $\pm$ 4.71%).
- The families with the highest relative abundance *Lachnospiraceae* (27.32 $\pm$ 9.97%), *Bacteroidaceae* (14.64 $\pm$ 7.50%), *Oscillospiraceae* (12.58 $\pm$ 3.13%) and *Prevotellaceae* (5.37 $\pm$ 7.08%).
- The genera with the highest relative abundance were *Phocaeicola* (9.04 $\pm$ 5.80%), *Blautia* (8.58 $\pm$ 5.63%) and *Faecalibacterium* (7.32 $\pm$ 3.93%).

*B. After 24-week intervention*

After the intervention period (probiotic/placebo), no differentially abundant taxonomic markers (from phylum to genus) were detected between the groups compared to baseline data.

### 3.2 SKIN MICROBIOTA

*A. Baseline*

In the skin microbiota of the included alopecia areata patients in the clinical trial:

- The phyla with the highest relative abundance were Actinobacteria (53.22 $\pm$ 20.07%), Firmicutes (26.88 $\pm$ 17.06%) and Proteobacteria (14.75 $\pm$ 13.45%).
- The families with the highest relative abundance were *Propionibacteriaceae* (34.35 $\pm$ 24.22%), *Staphylococcaceae* (20.05 $\pm$ 19.01%), *Bifidobacteriaceae* (8.82 $\pm$ 16.71%).
- The genera with the highest relative abundance were *Cutibacterium* (34.33 $\pm$ 24.24%), *Staphylococcus* (19.77 $\pm$ 19.15%) and *Bifidobacterium* (8.01 $\pm$ 16.58%).

*B. After 24-week intervention*

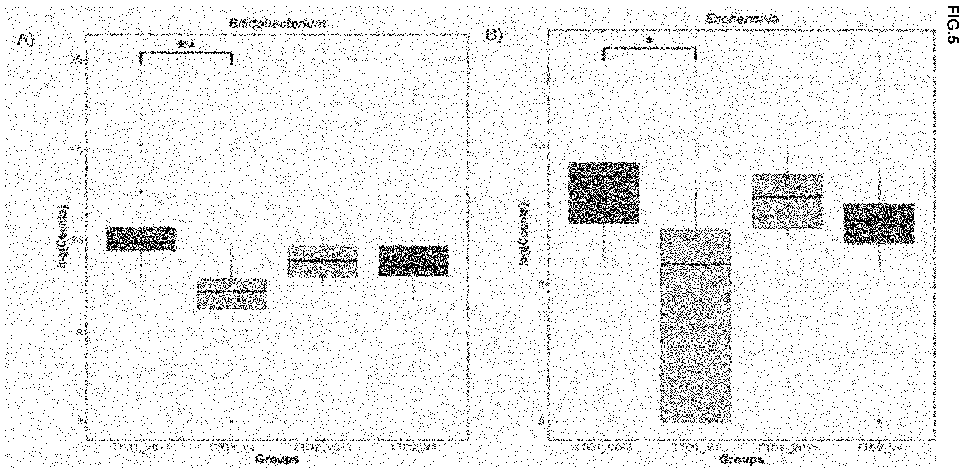

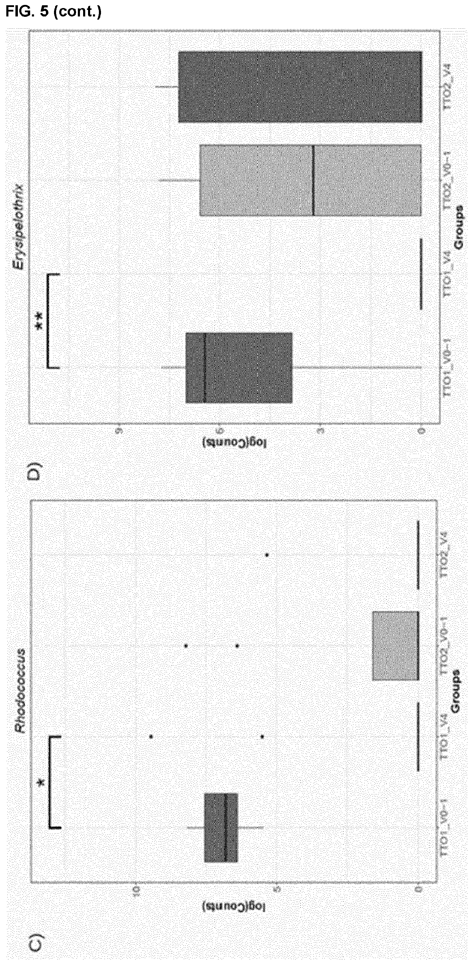

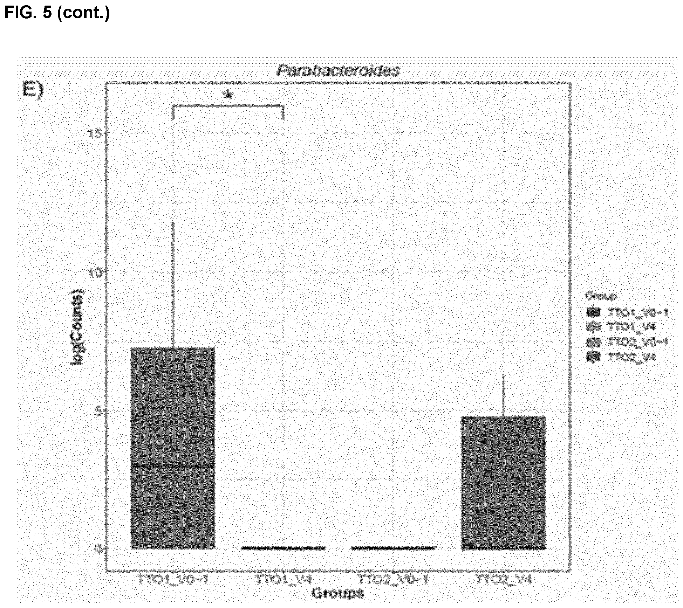

In the placebo group, no differentially abundant taxonomic markers were detected after the 24-weeks intervention period. Nevertheless, after the intervention with the probiotic treatment, changes at family and genus taxonomic levels were identified in the probiotic group, compared to the baseline visit (see Figure 5):

- At family level, *Bifidobacteriaceae, Erysipelotrichaceae* and *Nocardiaceae* were detected a lower relative abundance (reduced) compared to baseline visit.
- Within *Bifidobacteriaceae* family, *Bifidobacterium* genus was also significantly decreased.
- Within *Erysipelotrichaceae* family, there is *Erysipelothrix* genus, which was also significantly reduced.
- *Nocardiaceae* family is made up of different bacterial genera, including *Rhodococcus,* which was also significantly reduced.
- Other genera detected that were significantly decreased were: *Paraprevotella, Anaerobacillus, Escherichia-Shigella, Kineothrix, Parabacteroides, Lachnoclostridium, Alkalihalobacillus* and *Mediterraneibacter.*

In this study, all bacterial genera that have been reduced in the skin microbiota after the probiotic treatment of present invention with a greater improvement of clinical signs of alopecia areata compared with placebo are anaerobes or facultative anaerobes, such as *Bifidobacterium, Erysipelothrix, Paraprevotella, Rhodococcus, Anaerobacillus, Escherichia-Shigella, Kineothrix, Parabacteroides, Lachnoclostri-dium, Alkalihalobacillus* and *Mediterraneibacter.*

The probiotic composition of present invention therefore provides for a surprising and beneficial effect on the skin microbiome in reducing the amount of anaerobic or facultative anaerobic bacteria. On the other hand, these results together with the positive effect on alopecia aerate as shown in Example 1, indicate that there could be an association between a greater presence of these genera in the skin microbiota of patients with alopecia areata and the severity of the disease.

## References

(1) Hacini-Rachinel G., Gheit H., Le Luduec J.B., Dif F., Nancey S., Kaiserlian D. Oral probiotic control skin inflammation by acting on both effector and regulatory T cells. PLoS One. 2009; 4:e4903

(2) Bowe W.P., Logan A.C. Acne vulgaris, probiotics and the gut-brain-skin axis: back to the future? Gut Pathog. 2011;3:1

(3) Livingston M., Loach D., Wilson M., Tannock G.W., Baird M. Gut commensal Lactobacillus reuteri 100-23 stimulates an immunoregulatory response. Immunol Cell Biol. 2009;88:99 -102

(4) Benyacoub J., Bosco N., Blanchard C., Demont A., Phillippe D., Castiel-Higounenc I. Immune modulation property of Lactobacillus paracasei NCC2461 (ST11) strain and impact on skin defenses. Benef Microbes. 2014;5:129-136

(5) De Pessemier B, Grine L, Debaere M, Maes A, Paetzold B, Callewaert C. Gut-Skin Axis: Current Knowledge of the Interrelationship between Microbial Dysbiosis and Skin Conditions. Microorganisms. 2021; 9(2):353. doi: 10.3390/microorganisms9020353.

(6) Lousada MB, Lachnit T, Edelkamp J, Rouillé T, Ajdic D, Uchida Y, Di Nardo A, Bosch TCG, Paus R. Exploring the human hair follicle microbiome. Br J Dermatol. 2021; 184(5):802-815. doi: 10.1111/bjd .19461.

(7) Naik HB, Jo JH, Paul M, Kong HH. Skin Microbiota Perturbations Are Distinct and Disease Severity-Dependent in Hidradenitis Suppurativa. J Invest Dermatol. 2020; 140(4):922-925.e3. doi: 10.1016/j.jid.2019.08.445.

(8) Rebello D, Wang E, Yen E, Lio PA, Kelly CR. Hair Growth in Two Alopecia Patients after Fecal Microbiota Transplant. ACG Case Rep J. 2017; 4: e107. doi:10.14309/crj. 2017.107.

(9) Pinto D, Sorbellini E, Marzani B, Rucco M, Giuliani G, Rinaldi F. Scalp bacterial shift in Alopecia areata. PLoS One. 2019; 14(4):e0215206. doi: 10.1371/journal.pone.0215206.

## Claims

1. Probiotic composition for use in the treatment and/or prevention of cicatrical and/or non-cicatrical alopecia, preferably non-cicatrical alopecia, wherein the probiotic composition comprises at least one strain of *Lactobacillus* and at least one strain of *Bifidobacterium*.

2. The probiotic composition for the use according to claim 1, wherein

   (i) the at least one strain of *Lactobacillus* is selected from *L. rhamnosus, L. casei, L. paracasei* and *L. reuteri,* preferably wherein the strain is *L. rhamnosus,* most preferred *L.rhamnosus* CECT 30580; and/or
   (ii) the at least one strain of *Bifidobacterium* is selected from B. *animalis subspecies lactis* and *B. longum,* preferably wherein the strain is B. *longum,* most preferred B. *longum* CECT 30616.

3. The probiotic composition for the use according to claims 1 or 2, wherein

   (i) the cicatrical alopecia is selected from Lichen planopilaris (LPP), Lymphocytic cicatrical alopecia, such as Central centrifugal cicatricial alopecia (CCCA), Discoid lupus erythematous (DLE), Frontal fibrosing alopecia (FFA), Graham-Little syndrome (GLS), Pseudopelade of Brocq Follicular mucinosis (FM) and Keratosis follicularis spinulosa decalvans (KFSD), Neutrophilic cicatrical alopecia, such as Folliculitis decalvans (FD) and Dissecting cellulitis of the scalp (DCS), Mixed cicatrical alopecia, such as Acne keloidalis (AK), Acne necrotica (AN), Erosive pustular dermatosis of the scalp (EPDS), or any combinations thereof; and/or

(ii) the non-cicatrical alopecia is selected from Androgenetic Alopecia (AGA) and Alopecia Areata (AA).

4. The probiotic composition for the use according to claims 1 to 3, wherein the probiotic composition is a pharmaceutical composition or a nutritional composition.

5. The probiotic composition for the use according to claim 4, wherein the pharmaceutical composition comprises a pharmaceutically acceptable carrier and/or an excipient.

6. The probiotic composition for the use according to claims 4 or 5, wherein the pharmaceutical composition is formulated for administration in a solid formulation, wherein the solid formulation is selected from the group consisting of tablets, lozenges, sweets, chewable tablets, chewing gum, capsules, sachets, powders, granules, coated particles and/or tablets, gastro-resistant tablets and/or capsules and dispersible strips and/or films.

7. The probiotic composition for the use according to claims 4 or 5, wherein the pharmaceutical composition is formulated for administration in a liquid formulation, wherein the liquid formulation is selected from the group consisting of oral solutions, suspensions, emulsions and syrups.

8. The probiotic composition for the use according to claim 4, wherein the nutritional composition is a food or a nutritional supplement.

9. The probiotic composition for the use according to claim 8, wherein the said food is selected from the group consisting of fruit or vegetable juices, ice cream, milk, yogurt, cheese, fermented milk, milk powder, infant formula, cereals, baked goods, milk-based products, meat products and beverages.

10. The probiotic composition for the use according to claim 8, wherein the said nutritional supplement is selected from the group consisting of Biotin, Selenium, Zinc, Copper, Lutein or combinations thereof.

11. The probiotic composition for the use according to any of claims 1 to 10, wherein the composition further comprises further microorganisms selected from the group consisting of *Lactobacillus sp., Bifidobacterium sp., Kluyveromyces sp., Bacillus sp.,* and combinations thereof.

12. The probiotic composition for the use according to any of claims 1 to 11, wherein the probiotic composition is provided as a single dosage form.

13. The probiotic composition for the use according to claim 12, wherein the total concentration of microorganisms of the *Lactobacillus* and *Bifidobacterium* strains in the single dosage form is between about $10^3$ and $10^{15}$ cfu/dose, preferably between about $10^6$ and $10^{12}$ cfu/dose, more preferably about $10^9$ cfu/dose.

14. The probiotic composition for the use according to any of claims 1 to 13, wherein

(i) the *Lactobacillus* concentration is at least between 30% and 70%, at least between 40% and 60%, at least between 45% and 55% with respect to the total concentration of microorganisms present in the composition; and/or
(ii) the *Bifidobacterium* concentration is at least between 30% and 70%, at least between 40% and 60%, at least between 45% and 55% with respect to the total concentration of microorganisms present in the composition.

15. A probiotic composition comprising a mixture of at least two probiotic strains, wherein at least one probiotic strain is a strain of *Lactobacillus* and at least one probiotic strain is a strain of *Bifidobacterium,* wherein

(i) the at least one strain of *Lactobacillus* is selected from *L. rhamnosus, L. casei, L. paracasei* and *L. reuteri,* preferably wherein the strain is *L. rhamnosus,* most preferred *L.rhamnosus* CECT 30580;
(ii) the at least one strain of *Bifidobacterium* is selected from B. *animalis subspecies lactis* and *B. longum,* preferably wherein the strain is B. *longum,* most preferred B. *longum* CECT 30616.

FIG.1

**FIG.2**

**FIG.3**

**A)**

| Activity signs | Inactive disease | Signs of capillary repopulation |
|---|---|---|
| - Black dots<br>- Hairs in exclamation mark<br>- Broken hairs<br>- *Tapered hairs*<br>- Pseudomoniletrix | - Yellow dots<br>- Fluffy hairs<br>- Empty follicular orifices | - Straight hairs in regrowth<br>- *Pigtail hairs*<br>- Fluffy hairs |

**B)**

| Sign | Meaning |
|---|---|
| **Black spots (cadaveric hairs)** | - Pigmented broken or fractured hairs at the level of the scalp; Observed in approximately 50% of patients.<br>- Activity sign; multiple black dots indicate worse prognosis.<br>- Not specific to AA. |
| **Broken or exclamation mark hairs (=peladic hairs)** | - Fractured hairs with the distal end frayed and thicker.<br>- Activity sign. |
| **Yellow dots** | - 60 % patients<br>- If regular distribution = marker of severity and long-term disease<br>- Non-specific (can also appear in AGA). |
| **Tapered hairs (= coudability hairs)** | - Hairs of normal length, but with a narrower proximal shaft (indicating activity at the base of the hair).<br>- Sign of activity, more frequent around the plates. |
| **Other non-specific findings** | - Fluffy hairs.<br>- Vertical hairs in repopulation.<br>- Zigzag hairs.<br>- *Pili torti*.<br>- Tricorrexis nodosa.<br>- Pseudomoniletrix.<br>- Honeycomb pattern.<br>- interfollicular vascular loops. |

**FIG.4**

| Throughout last week... | Very much | Much | Slightly | Not at all |
|---|---|---|---|---|
| 1. Have you **felt itching**, **pain** or **burning** in the scalp or has it been **sore**? | | | | |
| 2. Have you felt **embarrassed** or **self-conscious** because of your hair? | | | | |
| 3. Has your hair condition bothered you to do the **shopping** or take care of the **house** or the **garden**? | | | | |
| 4. Has the condition of your hair influenced the choice of **clothes** you wear? | | | | |
| 5. Has your hair condition influenced any **social** or **leisure** activities? | | | | |
| 6. Have you had difficulty playing **sports** due to your hair condition? | | | | |
| 7. Has your hair condition prevented you from **working** or **studying**? | ☐ Yes | | ☐ No | |
| If the answer is "No", how much difficulty has your hair condition caused you at work or in your studies? | ■ | | | |
| 8. Has your hair condition caused you difficulties with your **partner**, **close friends** or **family**? | | | | |
| 9. How much difficulty has your hair condition caused you in your **sexual life**? | | | | |
| 10. How much difficulty has your hair **treatment** caused you? | | | | |
| | ×3 | ×2 | ×1 | ×0 |
| | | | | |
| **TOTAL** | | | | |

**FIG.5**

FIG. 5 (cont.)

FIG. 5 (cont.)

Europäisches
Patentamt

European
Patent Office

Office européen
des brevets

# EUROPEAN SEARCH REPORT

**Application Number**

EP 22 38 2491

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 114 404 351 A (MEIMOUSSE BEIJING SCIENCE AND TECH LIMITED COMPANY) 29 April 2022 (2022-04-29) | 1-9, 11-15 | INV. A61P17/02 A61K35/745 A61K35/747 A61P17/10 A61P17/14 |
| Y | * par. 22, 42; claims 1, 2, 4, 5, 7, 10 * | 10 | |
| X | WO 2019/169179 A1 (SHAFER KIM [US]) 6 September 2019 (2019-09-06) | 1-9, 11-15 | |
| Y | * par. 50, 51, 56 60, 68, 90, 115, 186 * | 10 | |
| Y | ALMOHANNA HIND M. ET AL: "The Role of Vitamins and Minerals in Hair Loss: A Review", DERMATOLOGY AND THERAPY, vol. 9, no. 1, 13 December 2018 (2018-12-13), pages 51-70, XP055967617, ISSN: 2193-8210, DOI: 10.1007/s13555-018-0278-6 Retrieved from the Internet: URL:https://www.ncbi.nlm.nih.gov/pmc/articles/PMC6380979/pdf/13555_2018_Article_278.pdf> * p. 55, col. 2, par. 1; p. 60, col. 2, par. 3; p. 61, col. 1, par. 3 * | 10 | |
| Y | WO 2013/178065 A1 (WINERGEN SCIENCE AND TECHNOLOGY WEIFANG CO LTD [CN]) 5 December 2013 (2013-12-05) * Abstract * | 10 | TECHNICAL FIELDS SEARCHED (IPC) A61P A61K |
| Y | TW 201 031 423 A (YU CHONGXI [US]) 1 September 2010 (2010-09-01) * par. 593 * | 10 | |

The present search report has been drawn up for all claims

| Place of search | Date of completion of the search | Examiner |
|---|---|---|
| Munich | 5 October 2022 | Seregélyes, Csaba |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 38 2491

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2022

| Patent document cited in search report | | Publication date | Patent family member(s) | | Publication date |
|---|---|---|---|---|---|
| CN 114404351 | A | 29-04-2022 | NONE | | |
| WO 2019169179 | A1 | 06-09-2019 | NONE | | |
| WO 2013178065 | A1 | 05-12-2013 | CN | 102659823 A | 12-09-2012 |
| | | | WO | 2013178065 A1 | 05-12-2013 |
| TW 201031423 | A | 01-09-2010 | AU | 2009322112 A1 | 10-06-2010 |
| | | | AU | 2017201227 A1 | 16-03-2017 |
| | | | BR | PI0922263 A2 | 01-09-2020 |
| | | | CA | 2745206 A1 | 10-06-2010 |
| | | | CA | 3013497 A1 | 10-06-2010 |
| | | | CN | 102307857 A | 04-01-2012 |
| | | | CN | 107261149 A | 20-10-2017 |
| | | | CN | 107320732 A | 07-11-2017 |
| | | | CN | 108191846 A | 22-06-2018 |
| | | | CN | 111808011 A | 23-10-2020 |
| | | | CN | 113636965 A | 12-11-2021 |
| | | | EP | 2370406 A1 | 05-10-2011 |
| | | | JP | 6670223 B2 | 18-03-2020 |
| | | | JP | 6793215 B2 | 02-12-2020 |
| | | | JP | 2012511027 A | 17-05-2012 |
| | | | JP | 2015205912 A | 19-11-2015 |
| | | | JP | 2017025109 A | 02-02-2017 |
| | | | JP | 2017036327 A | 16-02-2017 |
| | | | JP | 2018188472 A | 29-11-2018 |
| | | | JP | 2019089833 A | 13-06-2019 |
| | | | KR | 20110098933 A | 02-09-2011 |
| | | | KR | 20170078863 A | 07-07-2017 |
| | | | KR | 20180103109 A | 18-09-2018 |
| | | | KR | 20200022525 A | 03-03-2020 |
| | | | KR | 20210123406 A | 13-10-2021 |
| | | | MX | 337730 B | 16-03-2016 |
| | | | MX | 362949 B | 27-02-2019 |
| | | | RU | 2011127186 A | 10-01-2013 |
| | | | RU | 2017130909 A | 05-02-2019 |
| | | | TW | 201031423 A | 01-09-2010 |
| | | | US | 2011269689 A1 | 03-11-2011 |
| | | | WO | 2010065936 A1 | 10-06-2010 |

EPO FORM P0459

For more details about this annex : see Official Journal of the European Patent Office, No. 12/82

# EP 4 279 131 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **KLINDWORTH A et al.** *Nucleic Acids Res,* 2013, vol. 41, e1
- **ZHANG J et al.** Joining the ends to reconstruct unique sequences using the program 'pear' v0.9.6. *Bioinformatics,* 2014, vol. 30 (5), 614-20
- **MARTIN M.** Elimination of sequencing adapters and capture primers from the hypervariable regions V3 and V4 with the program cutadapt version 1.9.1. *EM-Bnet.journal [S.L.,* 2010, vol. 17 (1), ISSN 2226-6089, 10-12
- **EDGAR RC et al.** *Elimination of chimeras produced by PCR using the UCHIME program,* 15 August 2011, vol. 27 (16), 2194-200
- **HACINI-RACHINEL G. ; GHEIT H. ; LE LUDUEC J.B. ; DIF F. ; NANCEY S. ; KAISERLIAN D.** Oral probiotic control skin inflammation by acting on both effector and regulatory T cells. *PLoS One,* 2009, vol. 4, e4903
- **BOWE W.P. ; LOGAN A.C.** Acne vulgaris, probiotics and the gut-brain-skin axis: back to the future?. *Gut Pathog.,* 2011, vol. 3, 1
- **LIVINGSTON M. ; LOACH D. ; WILSON M. ; TANNOCK G.W. ; BAIRD M.** Gut commensal Lactobacillus reuteri 100-23 stimulates an immunoregulatory response. *Immunol Cell Biol.,* 2009, vol. 88, 99-102

- **BENYACOUB J. ; BOSCO N. ; BLANCHARD C. ; DEMONT A. ; PHILLIPPE D. ; CASTIEL-HIGOUNENC I.** Immune modulation property of Lactobacillus paracasei NCC2461 (ST11) strain and impact on skin defenses. *Benef Microbes.,* 2014, vol. 5, 129-136
- **DE PESSEMIER B ; GRINE L ; DEBAERE M ; MAES A ; PAETZOLD B ; CALLEWAERT C.** Gut-Skin Axis: Current Knowledge of the Interrelationship between Microbial Dysbiosis and Skin Conditions. *Microorganisms,* 2021, vol. 9 (2), 353
- **LOUSADA MB ; LACHNIT T ; EDELKAMP J ; ROUILLÉ T ; AJDIC D ; UCHIDA Y ; DI NARDO A ; BOSCH TCG ; PAUS R.** Exploring the human hair follicle microbiome. *Br J Dermatol.,* 2021, vol. 184 (5), 802-815
- **NAIK HB ; JO JH ; PAUL M ; KONG HH.** Skin Microbiota Perturbations Are Distinct and Disease Severity-Dependent in Hidradenitis Suppurativa. *J Invest Dermatol.,* 2020, vol. 140 (4), 922-925, e3
- **REBELLO D ; WANG E ; YEN E ; LIO PA ; KELLY CR.** Hair Growth in Two Alopecia Patients after Fecal Microbiota Transplant. *ACG Case Rep J.,* 2017, vol. 4, e107
- **PINTO D ; SORBELLINI E ; MARZANI B ; RUCCO M ; GIULIANI G ; RINALDI F.** Scalp bacterial shift in Alopecia areata. *PLoS One.,* 2019, vol. 14 (4), e0215206